# EUROPÄISCHE PATENTSCHRIFT

(11) **EP 3 947 615 B1**
(45) Veröffentlichungstag und Bekanntmachung des Hinweises auf die Patenterteilung: **05.04.2023**
(21) Anmeldenummer: 20710075.1
(22) Anmeldetag: 28.02.2020
(51) Int. Cl.: C11D 1/02, C11D 1/83, C11D 3/32, C11D 3/386, C11D 11/00

(54) **VERWENDUNG VON MANNANASE-ENZYM IN KOMBINATION MIT CATECHOLDERIVATEN**
USE OF A MANNANASE ENZYME IN COMBINATION WITH CATECHOL DERIVATIVES
UTILISATION DE L'ENZYME MANNANASE EN ASSOCIATION AVEC DES DÉRIVÉS DE CATÉCHOL

(30) Priorität: 04.04.2019 DE 102019204792
(43) Veröffentlichungstag der Anmeldung: 09.02.2022
(73) Patentinhaber: Henkel AG & Co. KGaA, 40589 Düsseldorf (DE)
(72) Erfinder: BERGMANN-DALKILIC, Simone, 40629 Düsseldorf (DE); JOB, Mareile, 51375 Leverkusen (DE); KROPF, Christian, 40724 Hilden (DE); MEIER, Frank, 40589 Düsseldorf (DE); PEGELOW, Ulrich, 40597 Düsseldorf (DE)
(86) Internationale Anmeldenummer: PCT/EP2020/055337
(87) Internationale Veröffentlichungsnummer: WO 2020/200600

(56) Entgegenhaltungen:
- WO-A1-2016/074936
- WO-A1-2018/184767
- US-A- 5 049 280

## Beschreibung

Die vorliegende Erfindung betrifft ein Waschmittel, bevorzugt ein Flüssigwaschmittel, welches mindestens eine Catecholverbindung, mindestens ein Tensid, und mindestens ein Mannanase-Enzym umfasst. Ferner betrifft die vorliegende Erfindung die Verwendung des Waschmittels zum Entfernen von bleichbaren Anschmutzungen sowie ein Verfahren zum Waschen von Textilien unter Verwendung des Waschmittels.

Während die Formulierung pulverförmiger, Bleichmittel enthaltender Wasch- und Reinigungsmittel heute keinerlei Probleme mehr bereitet, stellt die Formulierung stabiler flüssiger, Bleichmittel enthaltender Wasch- und Reinigungsmittel nach wie vor ein Problem dar. Aufgrund des üblicherweise Fehlens des Bleichmittels in flüssigen Wasch- und Reinigungsmitteln werden solche Anschmutzungen, die normalerweise insbesondere aufgrund der enthaltenen Bleichmittel entfernt werden, entsprechend häufig nur in unzureichender Weise entfernt. Ein ähnliches Problem besteht auch für Bleichmittel-freie Color-Waschmittel, bei denen das Bleichmittel weggelassen wird, um die Farbstoffe im Textil zu schonen und deren Ausbleichen zu verhindern. Bei fehlendem Bleichmittel kommt erschwerend hinzu, dass anstatt der Entfernung der sogenannten bleichbaren Anschmutzungen, die normalerweise durch den Einsatz von Bleichmittel auf Persauerstoffbasis wenigstens anteilsweise oxidativ zersetzt werden, aufgrund des Waschvorgangs häufig im Gegenteil sogar eine Intensivierung und/oder Verschlechterung der Entfernbarkeit der Anschmutzung herbeigeführt wird, was nicht zuletzt auf initiierte chemische Reaktionen zurückzuführen sein dürfte, die beispielsweise in der Polymerisierung bestimmter in den Anschmutzungen enthaltener Farbstoffe bestehen können.

Derartige Probleme treten insbesondere bei Anschmutzungen auf, die polymerisierbare Substanzen enthalten. Bei den polymerisierbaren Substanzen handelt es sich vor allem um polyphenolische Farbstoffe, vorzugsweise um Flavonoide, insbesondere aus der Klasse der Anthocyanidine oder Anthocyane. Die Anschmutzungen können insbesondere durch Lebensmittelprodukte oder Getränke verursacht worden sein, die entsprechende Farbstoffe enthalten. Bei den Anschmutzungen kann es sich insbesondere um Flecken von Früchten oder Gemüse oder auch Rotweinflecken handeln, die insbesondere polyphenolische Farbstoffe, vor allem solche aus der Klasse der Anthocyanidine oder Anthocyane, enthalten.

Aus der internationalen Patentanmeldung WO 2011/023716 A1 ist die Verwendung von Gallussäureestern wie Propylgallat in Wasch- und Reinigungsmitteln zur verbesserten Entfernung von Anschmutzungen, die polymerisierbare Substanzen enthalten, bekannt.

Die internationale Patentanmeldung WO 2013/092263 A1 betrifft die Verbesserung der Leistung von Wasch- und Reinigungsmitteln durch den Einsatz von Oligohydroxybenzoesäureamiden.

Die deutschen Patentanmeldungen DE 102016214660 A1 und DE 102014222833 betreffen die Verwendung von Dihydroxyterephthalsäurederivaten in Wasch- und Reinigungsmitteln zur Verbesserung der Wasch- oder Reinigungsleistung. Die Wasch- und Reinigungsmittel dieser Veröffentlichungen weisen einen alkalischen pH-Wert auf.

Es hat sich gezeigt, dass Lebensmittelprodukte, die neben den polymerisierbaren Substanzen zusätzlich Verdickungsmittel auf Polysaccharid-Basis enthalten, wie beispielsweise Schokoladenpudding, zu sehr schwer entfernbaren Anschmutzungen führen.

Die Aufgabe der vorliegenden Erfindung war es, eine Tensidzusammensetzung bereitzustellen, welche Anschmutzungen (insbesondere Anschmutzungen, die durch Lebensmittelprodukte hervorgerufen wurden, welche polymerisierbare Substanzen und Verdickungsmittel auf Polysaccharid-Basis enthalten) in hervorragendem Maße entfernt. Dabei soll eine gute Effizienz der Komponenten vorliegen und ein sehr gutes Waschergebnis, insbesondere ein hervorragender Weissgrad, erzielt werden.

Die Erfinder der vorliegenden Erfindung haben diesbezüglich herausgefunden, dass die Kombination spezieller Catecholverbindungen mit Mannanase zu einer verbesserten Fleckentfernung führt, insbesondere wenn die zu entfernende Anschmutzung durch Lebensmittelprodukte hervorgerufen wurde, die polymerisierbare Substanzen und Verdickungsmittel auf Polysaccharid-Basis enthalten.

Ein erster Gegenstand der vorliegenden Erfindung ist daher eine Tensidzusammensetzung, insbesondere als Textilwaschmittel, welche
- mindestens eine Catecholverbindung der Formel (I) 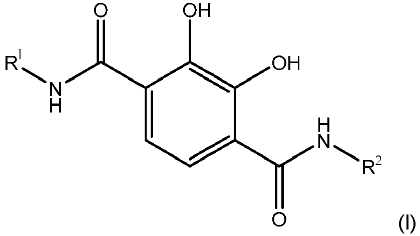 wobei
   R¹ und R² unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R^{'}R^{"}R^{‴} X, wobei n = 1 bis 10, R^{'}, R^{"} und R^{‴} unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen;
- mindestens ein Tensid, bevorzugt bezogen auf das Gesamtgewicht der Tensidzusammensetzung in einer Gesamtmenge von 2 bis 70 Gew.-%, insbesondere von 10 bis 65 Gew.-%, besonders bevorzugt von 15 bis 60 Gew.-%;
- mindestens ein Mannanase-Enzym;
- gegebenenfalls weitere Aktivstoffe; und
- Wasser bezogen auf das Gesamtgewicht der Tensidzusammensetzung von 1 bis 50 Gew.%, bevorzugt 2 bis 30 Gew.%, besonders bevorzugt 3 bis 25 Gew.%, ganz besonders bevorzugt 5 bis 25 Gew.%, insbesondere 5 bis 35 Gew.%, bevorzugt 5 bis 30 Gew.%, besonders bevorzugt 5 bis 25 Gew.%, ganz besonders bevorzugt 8 bis 15 Gew.%, bezogen auf das Gesamtgewicht der Tensidzusammensetzung umfasst.

Der Wassergehalt wie hierin definiert bezieht sich auf den mittels der Karl Fischer Titration ermittelten Wassergehalt (Angewandte Chemie 1935, 48, 394-396; ISBN 3-540-12846-8 Eugen Scholz).

"Flüssig", wie hierin in Bezug auf erfindungsgemäße Zusammensetzungen verwendet, schließt alle bei Standardbedingungen (20 °C, 1013 mbar) fließfähigen Zusammensetzungen in Form von Flüssigkeiten ein und erfasst insbesondere Flüssigkeiten, Gele und pastöse Zusammensetzungen. Insbesondere schließt der Begriff auch Nicht-Newtonsche Flüssigkeiten, die eine Fließgrenze besitzen, ein. Körnige Gemenge (fließfähige Feststoffe wie z.B. Pulver oder Granulatgemenge) sind bekanntermaßen keine Flüssigkeiten und daher nicht umfasst.

Alle im Zusammenhang mit den hierin beschriebenen Bestandteilen des Waschmittels angegeben Mengenangaben beziehen sich, sofern nichts anderes angegeben ist, auf Gew.% jeweils bezogen auf das Gesamtgewicht der Zusammensetzung. Desweiteren beziehen sich derartige Mengenangaben, die sich auf mindestens einen Bestandteil beziehen, immer auf die Gesamtmenge dieser Art von Bestandteil, die im Waschmittel enthalten ist, sofern nicht explizit etwas anderes angegeben ist. Das heißt, dass sich derartige Mengenangaben, beispielsweise im Zusammenhang mit "mindestens einem nichtionischen Tensid", auf die Gesamtmenge von nichtionischen Tensiden die im Waschmittel enthalten ist, beziehen.

"Mindestens ein", wie hierin verwendet, bezieht sich auf 1 oder mehr, beispielsweise 1, 2, 3, 4, 5, 6, 7, 8, 9 oder mehr. Im Zusammenhang mit Bestandteilen der hierin beschriebenen Zusammensetzungen bezieht sich diese Angabe nicht auf die absolute Menge an Molekülen sondern auf die Art des Bestandteils. "Mindestens ein nichtionisches Tensid" bedeutet daher beispielsweise ein oder mehrere verschiedene nichtionische Tenside, d.h. eine oder mehrere verschiedene Arten von nichtionischen Tensiden. Zusammen mit Mengenangaben beziehen sich

die Mengenangaben auf die Gesamtmenge der entsprechend bezeichneten Art von Bestandteil, wie bereits oben definiert.

"Im Wesentlichen frei von" wie hierin verwendet, bedeutet, dass die jeweilig betroffene Verbindung (z.B. Übergangsmetallion) bezogen auf das Gewicht der diese Verbindung enthaltene Komponente (z.B. Abtönfarbstoffe) oder Zusammensetzung (z.B. Waschmittel) in weniger als 0,01 Gew.-%, bevorzugt 0,001 Gew.-%, stärker bevorzugt 0,0001 Gew.-%, am stärksten bevorzugt gar nicht in der jeweiligen Komponente oder Zusammensetzung enthalten ist.

Sind Verbindungen in der vorliegenden Erfindung als substituiert beschrieben, so sind die möglichen Substituenten dem Fachmann bekannt. Insbesondere bevorzugt, falls nicht explizit anders angegeben, sind die Substituenten ausgewählt aus -F, -Cl, -Br, -I, -OH, =O, -OR', -NH₂, -NHR¹, -NR¹₂ und -COOR¹, wobei R¹ ein Alkylrest mit 1 bis 10 Kohlenstoffatomen ist.

Gemäß der vorliegenden Erfindung können mehrere Zusammensetzungen, dann als Komponenten bezeichnet, räumlich voneinander getrennt vorliegen.

In der vorliegenden Erfindung werden die Begriffe Waschmittel und Zusammensetzung synonym verwendet.

Betrifft die Erfindung ein Mehrkomponenten-Waschmittel, so können die weiteren Komponenten übliche Reinigungsmittel sein oder eine Zusammensetzung gemäß der vorliegenden Erfindung sein, die weitere Komponenten können auch fest sein oder als Granulat vorliegen. Bevorzugt ist, dass alle Komponenten flüssig sind.

Sind weitere flüssige Komponenten vorhanden, so können diese wie K1 oder K2 definiert sein.

Sind in der vorliegenden Erfindung Molekülmassen oder relative Molekülmassen oder Molmassen beschrieben, so handelt es sich, außer explizit anders angegeben um das zahlenmittlere Molekulargewicht M_{N}, welches mittels Gelpermeationschromatographie unter Verwendung von Polystyrolstandards bestimmt werden kann.

Vorzugsweise weisen Verbindungen der allgemeinen Formel (I) eine Löslichkeit in vollentsalztem Wasser von pH 4 bei 20°C von mindestens 10 mg/l, insbesondere mindestens 20 mg/l auf.

Dabei ist es bevorzugt, wenn bezogen auf das Gesamtgewicht der Zusammensetzung die mindestens eine Catecholverbindung gemäß Formel (I) in einer Gesamtmenge von 0,1 Gew.-% bis 90 Gew.-%, insbesondere von 0,5 Gew.-% bis 50 Gew.-%, bevorzugt von 1,0 Gew.-% bis 40 Gew.-%, weiter bevorzugt von 2,5 Gew.-% bis 20 Gew.-%, enthalten ist.

Die Kohlenwasserstoffreste mit 1 bis 20 Kohlenstoffatomen in Formel (I) können linear oder verzweigt, gesättigt oder ungesättigt, cyclisch oder alicyclisch oder aromatisch sein.

Bevorzugt eignen sich Zusammensetzungen, die mindestens eine Catecholverbindungen der Formel (I) enthalten, bei der die Reste R¹ und R² unabhängig voneinander stehen für eine Alkylgruppe, (wie Methyl, Ethyl, n-Propyl oder i-Propyl) eine Alkoxyalkylgruppe (wie Methoxyethyl, Methoxypropyl, (2-Methoxy)-ethoxyethyl, Ethoxyethyl, Ethoxypropyl oder (2-Ethoxy)-ethoxyethyl), eine Hydroxyalkylgruppe (wie 2-Hydroxyethyl, 3-Hydroxypropyl, 2-Hydroxypropyl, 1,2-Dihydroxypropyl), eine Hydroxyalkyloxyalkyl-Gruppe (wie 2-Hydroxyethoxyethyl), (N-Hydroxyethyl)-aminoethyl, (N-Methoxyethyl)-aminoethyl oder (N-Ethoxyethyl)-aminoethyl, oder eine aromatische Gruppe (wie Phenyl oder Benzyl).

Bevorzugte Alkylgruppen in Formel (I) sind lineare (C₁-C₁₀)-Alkylgruppen oder verzweigte (C₃-C₁₀)-Alkylgruppen, C₅-C₆-Cycloalkyl.

Es ist erfindungsgemäß bevorzugt, wenn in Formel (I) die Reste R¹ und R² gleich sind.

Weitere bevorzugte ausgewählte Verbindungen aus der Gruppe der Catechole ist mindestens eine Verbindung der allgemeinen Formel (I-a),

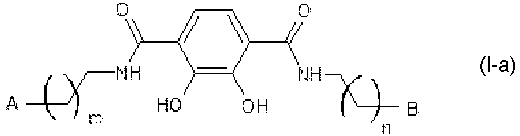

in der m und n unabhängig voneinander für 0 bis 5 und A und B unabhängig voneinander für ein Wasserstoffatom, -NR¹R², -N⁺R¹R²R³ X⁻, stehen sowie R¹, R² und R³ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen.

Unter den Verbindungen der allgemeinen Formel (I-a) sind solche bevorzugt, in denen A und B gleich sind.

X⁻ wird vorzugsweise aus der Gruppe umfassend Lactat, Citrat, Tartrat, Succinat, Perchlorat, Tetrafluoroborat, Hexafluorophosphat, Alkylsulfonat, Alkylsulfat, Hydrogensulfat, Sulfat, Dihydrogenphosphat, Hydrogenphosphat, Phosphat, Isocyanat, Rhodanid, Nitrat, Fluorid, Chlorid, Bromid, Hydrogencarbonat und Carbonat sowie Mischungen aus mindestens zweien von diesen ausgewählt, wobei der Ladungsausgleich bei Anwesenheit mehrwertiger Anionen durch die Anwesenheit entsprechend mehrerer kationischer Grundgerüste der allgemeinen Formel (I) oder gegebenenfalls durch die Anwesenheit zusätzlicher Kationen wie Natrium- oder Ammoniumionen gewährleistet werden kann. Es ist erfindungsgemäß bevorzugt, wenn gemäß Formel (I-a) A und B für ein Wasserstoffatom stehen.

Höchst bevorzugte Catechol-Verbindungen der Formel (I) sind die Verbindungen der Formeln (I-b) und/oder (I-c).

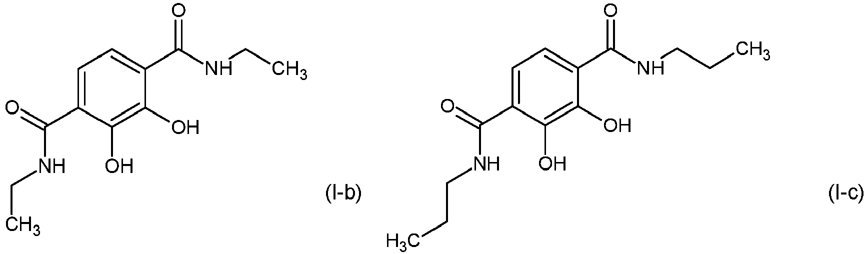

Die erfindungsgemäße Zusammensetzung enthält ferner zwingend mindestens ein Tensid. Dabei ist es wiederum besonders bevorzugt, wenn bezogen auf das Gewicht der erfindungsgemäßen Zusammensetzung Tensid in einer Gesamtmenge von 10 bis 70 Gew.-%, insbesondere 20 bis 65 Gew.-%, ganz besonders bevorzugt 30 bis 65 Gew.-% insbesondere 30 bis 60 Gew.-% enthalten ist.

Zur Gruppe der Tenside werden die nichtionischen, die anionischen, die kationischen und die amphoteren Tenside gezählt. Erfindungsgemäß kann die Zusammensetzung eines oder mehrere der genannten Tenside umfassen. Besonders bevorzugt umfasst sie wenigstens ein oder mehrere Aniontenside (anionische Tenside), welche vorzugsweise in einer Gesamtmenge von 15 bis 50 Gew.-%, insbesondere von 20 bis 40 Gew.-%, jeweils bezogen auf das Gewicht der Zusammensetzung, enthalten sind.

Das wenigstens eine anionische Tensid ist bevorzugt ausgewählt aus der Gruppe umfassend C₉-C,s-Alkylbenzolsulfonaten, Olefinsulfonaten, C₁₂-C₁₈-Alkansulfonaten, Estersulfonaten, Alk(en)ylsulfaten, Fettalkohohlethersulfaten und Mischungen daraus. Es hat sich gezeigt, dass sich diese Sulfonat- und Sulfat-Tenside besonders gut zur Herstellung stabiler flüssiger Zusammensetzungen eignen. Tensidzusammensetzungen, die als anionisches Tensid C₉-C₁₃-Alkylbenzolsulfonate und Fettalkoholethersulfate umfassen, weisen besonders gute, dispergierende Eigenschaften auf. Als Tenside vom Sulfonat-Typ kommen dabei vorzugsweise C₉-C₁₃-Alkylbenzolsulfonate, Olefinsulfonate, das heißt Gemische aus Alken- und Hydroxyalkansulfonaten sowie Disulfonaten, wie man sie beispielsweise aus C₁₂-C₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch C₁₂-C₁₈-Alkansulfonate und die Ester von α-Sulfofettsäuren (Estersulfonate), zum Beispiel die α-sulfonierten Methylester der hydrierten Kokos-, Palmkern- oder Talgfettsäuren.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate sind geeignete anionische Tenside.

Als Alk(en)ylsulfate werden bevorzugt die Salze der Schwefelsäurehalbester der Fettalkohole mit 12 bis 18 C-Atomen, beispielsweise aus Kokosfettalkohol, Talgfettalkohol, Lauryl-, Myristyl-, Cetyl- oder Stearylalkohol oder der Oxo-Alkohole mit 10 bis 20 C-Atomen und diejenigen Halbester sekundärer Alkohole dieser Kettenlängen bevorzugt. Aus waschtechnischem Interesse sind die Alkylsulfate mit 12 bis 16 C-Atomen und Alkylsulfate mit 12 bis 15 C-Atomen sowie Alkylsulfate mit 14 und 15 C-Atomen bevorzugt. Auch 2,3-Alkylsulfate sind geeignete anionische Tenside.

Auch Fettalkoholethersulfate, wie die Schwefelsäuremonoester der mit 1 bis 6 Mol Ethylenoxid ethoxylierten geradkettigen oder verzweigten C₇-C₂₁-Alkohole, wie 2-Methyl-verzweigte C9-11-Alkohole mit im Durchschnitt 3,5 Mol Ethylenoxid (EO) oder C12-18-Fettalkohole mit 1 bis 4 EO, sind geeignet. Bevorzugt sind Alkylethersulfate mit der Formel (A-1)

R¹-O-(AO)ₙ-SO₃⁻ X⁺ (A-1)

In dieser Formel (A-1) steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ der Formel (A-1) sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ der Formel (A-1) sind abgeleitet von Fettalkoholen mit 12 bis 18 C-Atomen, beispielsweise von Kokosfettalkohol, Talgfettalkohol, Lauryl-, Myristyl-, Cetyl- oder Stearylalkohol oder von Oxoalkoholen mit 10 bis 20 C-Atomen.

AO steht in Formel (A-1) für eine Ethylenoxid- (EO) oder Propylenoxid- (PO) Gruppierung, vorzugsweise für eine Ethylenoxidgruppierung. Der Index n der Formel (A-1) ist eine ganze Zahl von 1 bis 50, vorzugsweise von 1 bis 20 und insbesondere von 2 bis 10. Ganz besonders bevorzugt ist n 2, 3, 4, 5, 6, 7 oder 8. X ist ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺,½ Mg²⁺,½ Ca²⁺,½ Mn²⁺, und deren Mischungen.

Besonders bevorzugte Waschmittel enthalten ein Alkylethersulfat ausgewählt aus Fettalkoholethersulfaten der Formel A-2 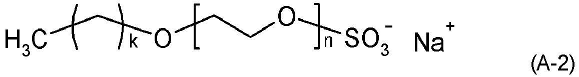 mit k = 11 bis 19, n = 2, 3, 4, 5, 6, 7 oder 8. Ganz besonders bevorzugte Vertreter sind Na Fettalkoholethersulfate mit 12 bis 18 C-Atomen und 2 EO (k = 11 bis 13, n = 2 in Formel A-1). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Es ist bevorzugt, dass die Zusammensetzung eine Mischung aus Sulfonat- und Sulfat-Tensiden enthält. In einer besonders bevorzugten Ausführungsform enthält die Zusammensetzung C9-13-Alkylbenzolsulfonate und gegebenenfalls zusätzlich Fettalkoholethersulfate als anionisches Tensid.

Es ist ganz besonders bevorzugt, wenn in der Zusammensetzung mindestens ein anionisches Tensid der Formel (A-3) enthalten ist, 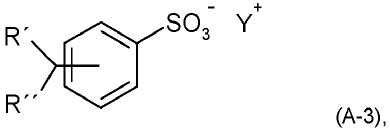 in der
R' und R" unabhängig H oder Alkyl sind und zusammen 9 bis 19, vorzugsweise 9 bis 15 und insbesondere 9 bis 13 C-Atome enthalten, und Y⁺ ein einwertiges Kation oder den n-ten Teil eines n-wertigen Kations (insbesondere Na⁺) bedeuten.

Zusätzlich zu dem anionischen Tensid kann die Zusammensetzung auch Seifen enthalten. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside sowie die Seifen können in Form ihrer Natrium-, Kalium- oder Magnesium- oder Ammoniumsalze vorliegen. Vorzugsweise liegen die anionischen Tenside in Form ihrer Ammoniumsalze vor, wobei sich das Ammonium-Ion von mindestens einem (C₂-C₆)-Alkanolamin ableitet. Weitere bevorzugte Gegenionen für die anionischen Tenside sind auch die protonierten Formen von Cholin, Triethylamin, Monoethanolamin, Triethanolamin oder Methylethylamin.

Die Zusammensetzung kann (bevorzugt gemeinsam mit mindestens einem anionischen Tensid) auch wenigstens ein nichtionisches Tensid aufweisen. Das nichtionische Tensid umfasst alkoxylierte Fettalkohole, alkoxylierte Fettsäurealkylester, Fettsäureamide, alkoxylierte Fettsäureamide, Polyhydroxyfettsäureamide, Alkylphenolpolyglycolether, Aminoxide, Alkylpolyglucoside und Mischungen daraus. Es ist wiederum besonders bevorzugt, wenn bezogen auf das Gewicht der erfindungsgemäßen Zusammensetzung nichtionisches Tensid in einer Gesamtmenge von 10 bis 40 Gew.-%, insbesondere von 15 bis 35 Gew.-%, enthalten ist.

Als nichtionisches Tensid werden vorzugsweise alkoxylierte, vorteilhafterweise ethoxylierte, insbesondere primäre Alkohole mit vorzugsweise 8 bis 18 C-Atomen und durchschnittlich 4 bis 12 Mol Ethylenoxid (EO) pro Mol Alkohol eingesetzt, in denen der Alkoholrest linear oder bevorzugt in 2-Stellung methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 5 bis 8 EO pro Mol Alkohol bevorzugt. Zu den bevorzugten ethoxylierten Alkoholen gehören beispielsweise C12-14-Alkohole mit 4 EO oder 7 EO, C9-11-Alkohol mit 7 EO, C13-15-Alkohole mit 5 EO, 7 EO oder 8 EO, C₁₂-C₁₈-Alkohole mit 5 EO oder 7 EO und Mischungen aus diesen. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO (Propylenoxid)-Gruppen zusammen im Molekül enthalten, sind erfindungsgemäß einsetzbar. Geeignet sind ferner auch eine Mischung aus einem (stärker) verzweigten ethoxylierten Fettalkohol und einem unverzweigten ethoxylierten Fettalkohol, wie beispielsweise eine Mischung aus einem C₁₆-C₁₈-Fettalkohol mit 7 EO und 2-Propylheptanol mit 7 EO. Insbesondere bevorzugt enthält das Waschmittel einen C₁₂-C₁₈-Fettalkohol mit 7 EO oder einen C₁₃-C₁₅-Oxoalkohol mit 7 EO als nichtionisches Tensid.

Besonders bevorzugt enthält die Zusammensetzung mindestens ein nichtionisches Tensid gemäß Formel (N-1)

R³-O-(XO)ₘ-H, (N-1)

in der
- R³: für einen linearen oder verzweigten C₈-C₁₈-Alkylrest, einen Arylrest oder Alkylarylrest,
- XO: unabhängig voneinander für eine Ethylenoxid- (EO) oder Propylenoxid- (PO) Gruppierung,
- m: für ganze Zahlen von 1 bis 50 stehen.

In der vorstehenden Formel (N-1) steht R¹ für einen linearen oder verzweigten, subtituierten oder unsubstituierten Alkylrest. In einer bevorzugten Ausführungsform der vorliegenden Erfindung ist R¹ ein linearer oder verzweigter Alkylrest mit 5 bis 30 C-Atomen, vorzugsweise mit 7 bis 25 C-Atomen und insbesondere mit 10 bis 19 C-Atomen. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecylresten und deren Mischungen, wobei die Vertreter mit gerader Anzahl an C-Atomen bevorzugt sind. Besonders bevorzugte Reste R¹ sind abgeleitet von Fettalkoholen mit 12 bis 19 C-Atomen, beispielsweise von Kokosfettalkohol, Talgfettalkohol, Lauryl-, Myristyl-, Cetyl- oder Stearylalkohol oder von Oxoalkoholen mit 10 bis 19 C-Atomen.

AO der Formel (N-1) ist eine Ethylenoxid- (EO) oder Propylenoxid- (PO) Gruppierung, vorzugsweise eine Ethylenoxidgruppierung. Der Index m der Formel (N-1) ist eine ganze Zahl von 1 bis 50, vorzugsweise 2 bis 20 und bevorzugt 2 bis 10. Insbesondere ist m 3, 4, 5, 6 oder 7. Die erfindungsgemäße Zusammensetzung kann Mischungen von nichtionischen Tensiden enthalten, die verschiedene Ethoxylierungsgrade aufweisen. Bevorzugt sind Tenside mit Alkoxylierungs-/Ethoxylierungsgraden von mindestens 5.

Zusammenfassend sind besonders bevorzugte Fettalkoholalkoxylate solche der Formel 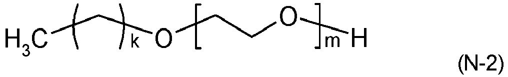 mit k = 9 bis 17, m = 3, 4, 5, 6 oder 7. Ganz besonders bevorzugte Vertreter sind Fettalkohole mit 10 bis 18 C-Atomen und mit 7 EO (k = 11-17, m = 7 in Formel N-2).

Solche Fettalkohol- oder Oxoalkoholethoxylate sind unter den Verkaufsbezeichnungen Dehydol^{®} LT7 (BASF), Lutensol^{®} AO7 (BASF), Lutensol^{®} M7 (BASF) und Neodol^{®} 45-7 (Shell Chemicals) erhältlich.

Die erfindungsgemäße Zusammensetzung enthält weiterhin zwingend mindestens ein Mannanase-Enzym.

Folgende Begriffe und Definitionen gelten zusätzlich im Rahmen der vorliegenden Anmeldung mit Blick auf die Definition von Enzymen.

"Variante" ist auf der Ebene der Proteine der zu "Mutante" entsprechende Begriff auf der Ebene der Nukleinsäuren. Bei den Vorgänger- oder Ausgangsmolekülen kann es sich um Wildtypenzyme handeln, das heißt solche, die aus natürlichen Quellen erhältlich sind. Es kann sich auch um Enzyme handeln, die an sich bereits Varianten darstellen, das heißt gegenüber den Wildtypmolekülen bereits verändert worden sind. Darunter sind beispielsweise Punktmutanten, solche mit Änderungen der Aminosäuresequenz, über mehrere Positionen oder längere zusammenhängende Bereiche, oder auch Hybridmoleküle zu verstehen, die aus einander ergänzenden Abschnitten verschiedener Wildtyp Enzyme zusammengesetzt sind.

Unter Aminosäureaustauschen sind Substitutionen einer Aminosäure gegen eine andere Aminosäure zu verstehen. Erfindungsgemäß werden solche Substitutionen unter Bezeichnung der Positionen, in der der Austausch erfolgt, gegebenenfalls kombiniert mit den betreffenden Aminosäuren im international gebräuchlichen Einbuchstabencode angegeben. "Austausch in Position 320" bedeutet beispielsweise, dass eine Variante in der Position, die in der Sequenz eines Referenzproteins die Position 320 aufweist, eine andere Aminosäure aufweist. Üblicherweise werden solche Austausche auf der DNA-Ebene über Mutationen einzelner Basenpaare durchgeführt (siehe oben). "R320K" bedeutet beispielsweise, dass das Referenzenzym an der Position 320 die Aminosäure Arginin aufweist, während die betrachtete Variante an der hiermit homologisierbaren Position über die Aminosäure Lysin verfügt. "320K" bedeutet, dass jede beliebige, das heißt in der Regel eine natürlicherweise vorgegebene Aminosäure an einer Position, die der Position 320 entspricht, gegen ein Lysin ersetzt ist, welches sich im vorliegenden Molekül eben an dieser Stelle befindet. "R320K, L" bedeutet, dass die Aminosäure Arginin in Position 320 gegen Lysin oder Leucin ersetzt ist. Und "R320X" bedeutet, dass die Aminosäure Arginin in Position 320 gegen eine prinzipiell beliebige andere Aminosäure ersetzt ist.

Grundsätzlich sind die mit der vorliegenden Anmeldung bezeichneten erfindungsgemäßen Aminosäureaustausche nicht darauf beschränkt, dass sie die einzigen Austausche sind, in denen sich die betreffende Variante von dem Wildtypmolekül unterscheidet. Es ist im Stand der Technik bekannt, dass sich die vorteilhaften Eigenschaften einzelner Punktmutationen einander ergänzen können. Somit umfassen Ausführungsformen der vorliegenden Erfindung alle Varianten, die neben anderen Austauschen gegenüber dem Wildtypmolekül auch die erfindungsgemäßen Austausche aufweisen.

Ferner spielt es prinzipiell keine Rolle, in welcher Reihenfolge die betreffenden Aminosäureaustausche vorgenommen worden sind, das heißt ob eine entsprechende Punktmutante erfindungsgemäß weiterentwickelt wird oder zunächst beispielsweise aus einem Wildtypmolekül eine erfindungsgemäße Variante erzeugt wird, die entsprechend anderer im Stand der Technik zu findender Lehren weiterentwickelt wird. Es können auch gleichzeitig in einem Mutageneseansatz mehrere Austausche vorgenommen werden, etwa erfindungsgemäße und andere zusammen.

Die Bestimmung der Identität von Nukleinsäure- oder Aminosäuresequenzen erfolgt erfindungsgemäß durch einen Sequenzvergleich. Solch ein Vergleich erfolgt dadurch, dass ähnliche Abfolgen in den Nukleotidsequenzen oder Aminosäuresequenzen einander zugeordnet werden. Dieser Sequenzvergleich erfolgt vorzugsweise basierend auf dem im Stand der Technik etablierten und üblicherweise genutzten BLAST-Algorithmus (vgl. beispielsweise Altschul, S. F., Gish, W., Miller, W., Myers, E. W. & Lipman, D. J. (1990) "Basic local alignment search tool." J. Mol. Biol. 215:403-410 , und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of Protein database search programs"; Nucleic Acids Res., 25, S. 3389-3402) und geschieht prinzipiell dadurch, dass ähnliche Abfolgen von Nukleotiden oder Aminosäuren in den Nukleinsäure- bzw. Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden üblicherweise mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen bzw. Algorithmen basieren. Häufig genutzt werden beispielsweise Clustal (vgl. beispielsweise Chenna et al. (2003): Multiple sequence alignment with the Clustal series of programs. Nucleic Acid Research 31, 3497-3500) oder T-Coffee (vgl. beispielsweise Notredame et al. (2000): T-Coffee: A novel method for multiple sequence alignments. J. Mol. Biol. 302, 205-217) sowie BLAST oder FASTA für die Datenbanksuche, beziehungsweise Programme, die auf diesen Programmen bzw. Algorithmen basieren. Im Rahmen der vorliegenden Erfindung werden Sequenzvergleiche und Alignments bevorzugt mit dem Computer-Programm Vector NTI^{®} Suite 10.3 (Invitrogen Corporation, 1600 Faraday Avenue, Carlsbad, Kalifornien, USA) mit den vorgegebenen Default-Parametern erstellt.

Solch ein Vergleich erlaubt eine Aussage über die Ähnlichkeit der verglichenen Sequenzen zueinander. Sie wird üblicherweise in Prozent Identität, das heißt dem Anteil der identischen Nukleotide oder Aminosäurereste an denselben bzw. in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch in Prozent Homologie oder Prozent Ähnlichkeit angegeben sein. Identitäts- und/oder Homologieangaben können über ganze Polypeptide oder Gene oder nur über einzelne Bereiche getroffen werden. Homologe bzw. identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide bzw. Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- bzw. Homologieangaben in der vorliegenden Anmeldung aber auf die Gesamtlänge der jeweils angegebenen Nukleinsäure- oder Aminosäuresäuresequenz.

Unter Fragmenten werden alle Polypeptide, Proteine oder Peptide verstanden, die kleiner sind als entsprechende Vergleichsproteine oder solche, die vollständig translatierten Genen entsprechen, und beispielsweise auch synthetisch erhalten werden können. Aufgrund ihrer Aminosäuresequenzen können sie den betreffenden vollständigen Vergleichsproteinen zugeordnet werden. Sie können beispielsweise gleiche räumliche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche (unter Umständen nur ein oder mehrere Aminosäuren). So ist es beispielsweise möglich, an den Termini oder in den Loops des Enzyms weitere einzelne Aminosäuren zu deletieren, ohne dass dadurch die enzymatische Aktivität verloren oder vermindert wird.

Alle Mengenangaben von Enzymen beziehen sich auf aktives Protein. Die Proteinkonzentration kann mit Hilfe bekannter Methoden, zum Beispiel dem BCA-Verfahren (Bicinchoninsäure; 2,2`-Bichinolyl-4,4'-dicarbonsäure) oder dem Biuret-Verfahren (Gornall et al., J. Biol. Chem. 177 (1948): 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al., J. Am. Chem. Soc. 88, 24 (1966): 5890-5913) erfolgen.

Im Allgemeinen können die in einer erfindungsgemäßen Zusammensetzung enthaltenen Enzyme an Trägerstoffe adsorbiert und/oder in Hüllsubstanzen eingebettet sein, um sie gegen vorzeitige Inaktivierung zu schützen. In den hierin beschriebenen Zusammensetzungen können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Erfindungsgemäße Zusammensetzungen können die erhaltenen Enzyme in jeder nach dem Stand der Technik etablierten Form zugesetzt werden. Hierzu gehören insbesondere die durch Granulation, Extrusion oder Lyophilisierung erhaltenen festen Präparationen, vorteilhafterweise möglichst konzentriert, wasserarm und/oder mit Stabilisatoren versetzt. In einer alternativen Darreichungsform können die Enzyme auch verkapselt werden, beispielsweise durch Sprühtrocknung oder Extrusion der Enzymlösung zusammen mit einem, vorzugsweise natürlichen Polymer oder in Form von Kapseln, beispielsweise solchen, bei denen die Enzyme wie in einem erstarrten Gel eingeschlossen sind, oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

In verschiedenen Ausführungsformen kann das erfindungsgemäße Mittel einen oder mehrere Enzymstabilisatoren aufweisen. Daher kann das erfindungsgemäße Mittel ferner einen Enzymstabilisator beispielsweise ausgewählt aus der Gruppe bestehend aus Natriumformiat, Natriumsulfat, niederen aliphatischen Alkoholen und Borsäure sowie deren Estern und Salzen enthalten. Natürlich können auch zwei oder mehr dieser Verbindungen in Kombination eingesetzt werden. Die Salze der genannten Verbindungen können auch in Form von Hydraten, wie beispielsweise Natriumsulfat Dekahydrat, eingesetzt werden.

Ein in der erfindungsgemäßen Zusammensetzung enthaltenes Mannanase-Enzym katalysiert im Rahmen seiner Mannanase-Aktivität die Hydrolyse von 1,4-beta-D-mannosidischen Bindungen in Mannanen, Galactomannanen, Glucomannanen und Galactoglucomannanen. Besagte erfindungsgemäße Mannanase-Enzyme werden gemäß Enzym Nomenklatur als E.C. 3.2.1.78 klassifiziert. Synonyme für dies erfindungsgemäßen Mannanase-Enzyme sind Galactomannanase, Mannan-endo-1,4-β-Mannosidase, β-Mannanase oder Endo-1,4-Mannanase.

Als "Mannane" bezeichnet man Mannose enthaltende Polysaccharide, die aus einem Mannose-Grundgerüst, in dem die Mannose-Einheiten über β-1,4-glykosidische Bindungen verknüpft sind, mit Seitenketten aus Galactose-Einheiten, die über α-1,6-glykosidische Verknüpfungen an das Grundgerüst angebunden sind. Als "Glucomannane" bezeichnet man Polysaccharide mit einem Rückgrat aus mehr oder weniger regelmäßig wechselnden β-1 ,4-glykosidisch verknüpften Mannose- und Glucose-Einheiten. Als "Galactomannane" und "Galactoglucomannane" bezeichnet man Mannane und Glucomannane mit α-1,6-glykosidisch verknüpften Galactose-Seitenketten.

Wie hierin verwendet, hat ein Enzym dann "Mannanase-Aktivität", wenn es mannanabbauende Eigenschaften aufweist. "Abbau" oder "Modifizierung", wie hierin verwendet, bedeutet, dass Mannose-Einheiten aus dem Mannan-Polysaccharid durch Mannanase hydrolysiert werden. Die mannanabbauende Aktivität der Polypeptide gemäß der vorliegenden Erfindung kann nach den im Stand der Technik bekannten Standardtestverfahren bestimmt werden. Beispiel 7 der WO 2018/184767 A1 zeigt ein Beispiel für eine Standardmethode zur Bestimmung der Mannanase-Aktivität. In einem weiteren Testverfahren wird beispielsweise eine Testlösung in Löcher mit 4 mm Durchmesser einer Agarplatte, enthaltend 0.2 Gew.-% AZGL Galactomannan (carob), i.e. Substrat für das endo-1,4-beta-D-Mannanase Essay, erhältlich unter Katalognummer I-AZGMA der Firma Megazyme (http://www.megazyme.com), eingebracht.

Die Mannanase-Aktivität eines Polypeptids bzw. Enzyms kann gemäß literaturbekannten Testmethoden bestimmt werden. Dabei wird beispielsweise eine Testlösung in Löcher mit 4 mm Durchmesser einer Agarplatte, enthaltend 0.2 Gew.-% AZGL Galactomannan (carob), i.e. Substrat für das endo-1,4-beta-D-Mannanase Essay, erhältlich unter Katalognummer I-AZGMA der Firma Megazyme (http://www.megazyme.com), eingebracht.

Kürzlich wurden die ersten in Wasch- und Reinigungsmittel stabilen Mannanasen gefunden. In dem Mannanasen die β-1,4-glykosidische(n) Bindung(en) zwischen Mannose-Einheiten aufbrechen und das Mannan so zu kleineren, besser wasserlöslichen Kohlenhydratfragmenten abbauen, helfen sie bei der Entfernung mannanhaltiger Flecken und reduzieren zudem das Risiko einer Wiederanlagerung.

Geeignete Mannanasen sind z.B. die *Bacillus subtilis* endo-β-mannanase (vgl. z.B. US 6060299 und WO 99/64573), *Bacillus sp.* I633 endo-β-mannanase (vgl. z.B. US6566114 und WO 99/64619), *Bacillus sp.* AAI12 endo-β-mannanase (vgl. z.B. US6566114 und WO 99/64619), *Bacillus sp.* AA349 endo-β-mannanase (vgl. z.B. US 6566114 und WO 99/64619), *Bacillus agaradhaerens* NCIMB 40482 endo-β-mannanase (vgl. z.B. US 6566114 und WO 99/64619), *Bacillus halodurans* endo-β-mannanase, *Bacillus clausii* endo-β-mannanase (vgl. z.B. US 6566114 und WO 99/64619), *Bacillus licheniformis* endo-β-mannanase (vgl. z.B. US 6566114 und WO 99/64619), *Humicola insolens* endo-β-mannanase (vgl. z.B. US 6566114 und WO 99/64619) und *Caldocellulosiruptor sp.* endo-β-mannanase (vgl. z.B. US 6566114 und WO 99/64619), und *Paenibacillus sp.* (WO 2017/079751 A1).

Bevorzugterweise enthalten die erfindungsgemäßen Zusammensetzungen bezogen auf das Gesamtgewicht der Zusammensetzung Mannanase-Enzym in einer Gesamtmenge von 0,01 bis 2,5 Gew.-%, insbesondere von 0,02 bis 1,0 Gew.-%, ganz besonders bevorzugt von 0,02 bis 0,7 Gew.-%.

Mannanase-Polypeptide aus Stämmen der *Thermoanaerobacter Gruppe,* wie *Caldicellulosiruptor,* sind erfindungsgemäß bevorzugt geeignet. Gleichfalls im Rahmen der Erfindung einsetzbar sind Mannanase-Polypeptide der Pilze *Humicola* oder *Scytalidium,* insbesondere der Species *Humicola insolens* oder *Scytalidium thermophilum.*

Es ist erfindungsgemäß besonders bevorzugt, wenn die erfindungsgemäßen Zusammensetzungen als Mannanase-Enzym mindestens ein Mannanase-Polypeptid aus gram-positiven alkalophilen Stämmen von *Bacillus* enthält, insbesondere ausgewählt aus mindestens einem Vertreter der Gruppe aus *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis, and Bacillus sp., besonders bevorzugt ausgewählt aus mindestens einem Vertreter der Gruppe aus Bacillus sp. 1633, Bacillus sp. AA112, Bacillus clausii, Bacillus agaradhaerens and Bacillus licheniformis.*

Ein bevorzugtes erfindungsgemäßes Mannanase-Enzym wird ausgewählt aus mindestens einem Vertreter aus der Gruppe, die gebildet wird aus
i) Polypeptiden, die eine Aminosäuresequenz umfassen, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%) Sequenzidentität zu dem Polypeptid gemäß SEQ ID No.1 (vgl. Sequenzprotokoll), und
ii) Polypeptiden, die ein Fragment von (i) sind.

Dabei ist es wiederum bevorzugt, wenn besagte bevorzugte Mannanase in einer Gesamtmenge von 0,01 bis 5,0 Gew.-%, insbesondere von 0,2 bis 2,0 Gew.-%, jeweils bezogen auf das Gesamtgewicht der Zusammensetzung, in der erfindungsgemäßen Zusammensetzung enthalten ist.

Unter dem jeweils oben unter (ii) definierten Fragment werden im Falle der bevorzugt einsetzbaren Mannanase und analog zu der eingangs erwähnten Definition alle Polypeptide, Proteine oder Peptide verstanden, die kleiner sind als die unter (i) fallenden Polypeptide oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche (unter Umständen nur ein oder mehrere Aminosäuren). So ist es beispielsweise möglich, an den Termini oder in den Loops des Enzyms weitere einzelne Aminosäuren zu deletieren, ohne dass dadurch die enzymatische Aktivität verloren oder vermindert wird. Besonders bevorzugt sind Deletionen von insbesondere 1, bis zu 2, bis zu 3, bis zu 4, bis zu 5, insbesondere bis zu 10, bevorzugt bis zu 20, insbesondere bevorzugt bis zu 30 Aminosäuren auf der N-terminalen Seite des Polypeptids (bevorzugt des Polypeptids der SEQ. ID NO.1). Dabei wird die enzymatische Aktivität der Mannanase bevorzugt nicht oder nur in einem geringen Maßen, insbesondere nur bis zu einer Reduktion von 15% der Aktivität des Ursprungsenzyms (besonders bevorzugt für Mannanase gemäß SeqlD No. 1), reduziert.

Besonders bevorzugt wird die Mannanase ausgewählt aus mindestens einem Vertreter aus der Gruppe, die gebildet wird aus Polypeptiden, die eine Aminosäuresequenz umfassen, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%) Sequenzidentität zu dem Polypeptid der Positionen 31 bis 490 gemäß SEQ ID No.1 (vgl. Sequenzprotokoll) aufweisen.

Ganz besonders bevorzugt weisen die Polypeptide eine Aminosäuresequenz auf, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%) Sequenzidentität zu dem Polypeptid der Positionen 31 bis 490 gemäß SEQ ID No.1 (vgl. Sequenzprotokoll).

Weiter bevorzugt weisen die Polypeptide eine Aminosäuresequenz auf, die mindestens 90% (zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%) Sequenzidentität zu dem Polypeptid der Positionen 31 bis 330 gemäß SEQ ID No.1 (vgl. Sequenzprotokoll)

Insbesondere bevorzugt ist sind Polypeptide, deren Aminosäuresequenz zu mehr als 99,0% mit der Sequenz gemäß SEQ ID No.1 identisch ist.

Ein bevorzugtes Mannanase-Enzym wird gemäß Anspruch 1 der WO 99/64619 offenbart, in der Beschreibung dieser WO-Druckschrift näher beschrieben und ist demnach ausgewählt aus mindestens einem Mannanase-Enzym, dass ausgewählt wird aus mindestens einem Vertreter aus der Gruppe, die gebildet wird aus
i) Polypeptiden, die durch den für das Mannanaseenzym kodierenden Teil der DNA-Sequenz kodiert werden können, die in das in Escherichia coli DSM 12197 vorliegende Plasmid kloniert ist,
ii) Polypeptiden, die eine Aminosäuresequenz umfassen, wie an den Positionen 33-340 von SEQ ID NO: 1 in WO 99/64619 gezeigt,
iii) Polypeptiden, die eine Aminosäuresequenz umfassen, wie an den Positionen 31-990 oder den Positionen 91-1470, jeweils von SEQ ID NO: 1 in WO 99/64619 gezeigt, oder
iv) Analoga der in (i) oder (ii) definierten Polypeptide, die mindestens 90% (und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) Sequenzidentität zu dem Polypeptid haben, oder ein Fragment von (i), (ii) oder (iii) sind.

Die im Rahmen der vorliegenden Erfindung unter SEQ ID No.1 beschriebene Sequenz entspricht der in der WO 99/64619 offenbarten Sequenz unter SEQ ID No.2.

Zur Auslegung der Merkmale der zuvor definierten, erfindungsgemäß bevorzugten Mannanase-Enzyme (*vide supra*) ist ausdrücklich auch die Gesamtoffenbarung der WO 99/64619 vollumfänglich heranzuziehen. Ebenso gelten die in der WO 99/64619 als bevorzugt genannten Mannanase-Enzyme im Sinne der erfindungsgemäßen Zusammensetzung als bevorzugt.

Eine weitere bevorzugte Ausführungsform der Erfindung enthält mindestens eine Mannanase aus *Paenibacillus sp.* oder eine der Varianten davon, die in WO 2017/079751 A1 beschrieben sind. Eine solche Mannanasevariante umfasst eine Aminosäuresequenz, die mindestens zwei Veränderungen aufweist, die ausgewählt sind aus
(i) mindestens einer Substitution an mindestens einer der Positionen, die aus 1, 2, 3, 4, 6, 10, 19, 28, 30, 38, 59, 60, 61, 62, 63, 66, 67, 68, 70, 71, 74, 75, 78, 80, 82, 93, 97, 103, 111, 124, 129, 131, 135, 136, 139, 143, 150, 167, 168, 184, 213, 214, 217, 225, 228, 235, 242, 244, 258, 259, 261, 283 und 284 ausgewählt sind, und
(ii) einer Insertion an Position 298, wobei die genannte Aminosäurepositionen den Positionen gemäß SEQ ID No. 2 dieser Anmeldung. SEQ ID No. 2 (entspricht SEQ ID No. 14 der WO 2017/079751 A1) entsprechen.

Zur Offenbarung der vorgenannten bevorzugten Mannanase aus *Paenibacillus sp.* oder eine deren Varianten wird ausdrücklich und vollinhaltlich auf die Offenbarung der WO 2017/079751 A1 verwiesen. Insbesondere gelten in diesem Zusammenhang für die Varianten zusätzlich die Definition für den Buchstabencode "Z" als Insertion oder Deletion in einer Stamm- oder Referenz-Aminosäuresequenz. Für eine Insertion relativ zu einer Stamm- oder Referenzsequenz, ist der Code "Z" auf der linken Seite einer Positionsnummer notiert und umfasst weiter eine Nummer (e.g., .01), welche vor dem Code der insertierten Aminosäure zur Indikation der Ordnung der Insertion positioniert ist. Beispielsweise die Insertion der Aminosäure Glutamin (Q) an der Position 298 wird wiedergegeben als "Z298.01Q"; die Insertion der Aminosäure X (wobei X jede beliebige Aminosäure ist) an Position 298 wird wiedergegeben als "Z298.01X"; und die Insertion von drei Aminosäuren Alanine (A), Serin (S) und Tyrosin (Y) zwischen Position 87 und 88 wird wiedergegeben als "Z87.01N / Z87.02S / Z87.03Y". Für eine Deletion wird der Code "Z" auf der rechten Seite der Positionsnummer angeordnet. Beispielsweise, die Deletion von Alanin (A) von der Position 100 wird als A100Z wiedergegeben. Eine Kombination einiger der oben genannten Insertionen und Deletionen wird beispielsweise wiedergegeben als: "G87S / Z87.01N / Z87.02S / Z87.03Y / A100Z".

In einer bevorzugten Ausführungsform umfasst eine solche Mannanasevariante mindestens zwei Veränderungen, die ausgewählt sind aus
(i) mindestens einer Substitution, die aus der aus M1V, M1L, A2S, T3R, G4S, Y6E, N10T, N10S, P19E, G28A, G28S, S30T, T38E, S59D, S59V, L60Q, Y61W, T62E, K63R, K63L, L66V, N67D, A68S, K70R, N71D, N74E, N74S, V75L, Q78D, Q78H, K80T, I82M, K93R, N97D, N97L, V103I, E111D, E111S, I124V, Y129M, T131A, T135L, A136L, D139M, K143Q, K143R, N150T, F167Y, P168A, P168S, Q184D, Q184L, N213A, K214I, A217P, G225C, G225P, T228V, Y235L, Q242L, K244L, S258D, G259P, N261Q, N261R, D283S, T284A und T284E bestehenden Gruppe ausgewählt ist, und
(ii) einer Insertion an Position Z298.01Q, wobei die genannte Aminosäurepositionen den Positionen gemäß SEQ ID No. 2 entsprechen.

In einer weiter bevorzugten Ausführungsform umfasst eine solche Mannanasevariante eine der folgenden Veränderungskombinationen: P19E-T38E-K63L-N71D-Y129M-Q184L-K244L-S258D-N261R; N67D-Y129M-P168S-Q184L-K244L-S258D-G259P; P19E-K63L-N67D-Q78D-K80T-N97D-Y129M-G225C-T228V-K244L; P19E-T38E-N67D-N97D-Y129M-P168S-Q184L-K244L-S258D-N261R; P19E-T38E-N67D-N71D-Q78D-K80T-N97D-Y129M-P168S-G225C-K244L-S258D-N261R; T38E-K63L-N71D-N97D-Y129M-Q184L-G225C-T228V-Q242L-K244L-S258D-N261R; P19E-K63L-N71D-N97D-Y129M-Q184L-G225C-K244L-S258D-G259P; N10T-T38E-S59V-L60Q-K63R-L66V-A68S-N74S-V75L-N97D-V103I-Y129M-F167Y-Q184L-A217P-G225C-Y235L-K244L-S258D-N261R-Z298.01Q; P19E-T38E-N67D-N71D-N97D-Y129M-F167Y-Q184L-A217P-K244L-S258D-N261R; T38E-K63L-N67D-Q78D-K80T-N97D-Y129M-P168S-Q184L-K244L-S258D-N261R; P19E-T38E-N67D-Y129M-P168S-Q184L-K244L-S258D-N261R; P19E-N67D-N97D-Y129M-P168S-Q184L-K244L; P19E-T38E-K63L-N71D-Y129M-P168S-G225C-T228V-K244L-S258D-N261R; P19E-T38E-N67D-N97D-Q184L-A217P-G225C-T228V-Y235L-K244L-S258D-N261R; N10T-P19EG28S-S30T-T38E-N67D-N71D-N97D-Y129M-P168S-Q184L-G225C-Y235L-K244L-S258D-N261R-Z298.01Q; P19E-T38E-S59V-L60Q-K63R-N67D-N97D-V103I-Y129M-K143Q-F167Y-Q184L-G225C-Y235L-K244L-S258D-N261R-Z298.01Q; P19E-T38E-N67D-N71D-Q78D-K80T-N97D-Y129M-P168S-G225C-T228V-K244L-S258D-N261R-Z298.01Q; P19E-T38E-S59V-L60Q-K63L-K70R-N71D-Q78D-K80T-N97D-E111D-Y129M-Q184L-G225C-T228V-Y235L-K244L-S258D-N261R-Z298.01Q; N10T-T38E-K63L-N71D-N97D-V103I-Y129M-F167Y-Q184L-G225C-T228V-Y235L-K244L-S258D-N261R-Z298.01 Q; N10T-P19E-T38E-N67D-Q78D-K80T-N97D-129M-K143Q-Q184L-A217P-G225C-T228V-Y235L-K244L-S258D-N261R-Z298.01Q; N10T-P19E-T38ES59V-L60Q-K63L-N97D-V103I-Y129M-F167Y-Q184L-G225C-Y235L-K244L-S258D-N261R-Z298.01Q; P19E-S30T-T38E-S59V-L60Q-K63R-N67D-N97D-V103I-Y129M-F167Y-Q184L-G225C-T228V-Y235L-K244L-S258D-N261R-Z298.01Q; P19E-S30T-T38E-S59V-L60Q-K63R-N67D-Q78D-K80T-N97D-I124V-Y129M-K143Q-F167Y-Q184L-G225C-Y235L-K244L-S258D-N261R-Z298.01Q; N10S-P19E-S30T-T38E-S59V-L60Q-K63L-N67D-Q78H-K80T-I82M-N97D-Y129M-K143Q-F167Y-Q184L-G225C-Y235L-K244L-S258D-N261R-Z298.01Q; N10T-P19E-S30T-T38E-S59V-L60Q-K63R-N67D-N97D-Y129M-K143Q-P168S-Q184L-G225C-T228V-Y235L-K244L-S258D-N261R-Z298.01Q; G4S-N10T-P19E-T38E-N67D-Q78D-K80T-N97D-Y129M-Q184L-G225C-T228V-Y235L-K244L-S258D-N261R-Z298.01Q; N10T-P19E-S30T-T38E-S59V-L60Q-K63L-K70R-N71D-Q78D-K80T-N97D-Y129M-T131A-F167Y-Q184L-G225C-Y235L-K244L-S258D-N261R-Z298.01Q; N10T-P19E-S30T-T38E-S59V-L60Q-K63L-K70R-N71D-Q78D-K80T-N97D-E111D-Y129M-P168S-Q184L-G225C-T228V-Y235L-K244L-S258D-N261R-Z298.01Q; P19ES30T-T38E-S59V-L60Q-K63R-N67D-N97D-Y129M-P168S-Q184L-K2141-G225C-Y235L-K244L-S258D-N261R-Z298.01Q; N10T-P19E-S30T-T38E-S59V-L60Q-K63R-N67D-N97D-Y129M-K143Q-P168S-Q184L-G225P-T228V-Y235L-K244L-S258D-N261R-Z298.01Q; M1V-P19E-S30T-T38E-T62E-N67D-N71D-Q78D-N97D-Y129M-K143R-F167Y-P168S-Q184L-G225C-Y235L-K244L-S258D-N261R-T284A-Z298.01Q; Y6E-N10T-P19E-G28S-S30T-T38E-K63L-N67D-N71D-N97D-E111S-Y129M-S135L-P168S-Q184L-G225C-T228V-Y235L-K244L-S258D-N261Q-D283S-Z298.01Q; N10T-P19E-S30T-T38E-S59V-L60Q-K63R-N67D-N71D-N97D-V103I-Y129M-K143Q-P168S-Q184L-G225P-T228V-Y235L-K244L-S258D-N261R-Z298.01Q; A2S-P19E-G28S-S30T-T38E-K63R-N67D-N71D-N74E-K93R-N97D-Y129M-N150T-P168S-Q184L-N213A-G225C-Y235L-K244L-S258D-N261Q-Z298.01Q; M1L-N10T-P19E-G28A-S30T-T38E-K63L-N67D-N71D-Q78D-N97D-Y129M-A136L-P168A-Q184L-N213A-G225C-Y235L-K244L-S258D-N261R-Z298.01Q; P19E-T38E-S59V-K63R-N67D-N97D-V103I-Y129M-F167Y-Q184L-G225C-T228V-Y235L-K244L-S258D-N261R-Z298.01Q; N10T-P19E-G28A-S30T-T38E-K63R-N67D-N97D-Y129M-Q184L-G225C-T228V-Y235L-K244L-S258D-N261R-Z298.01Q; T3R-N10T-P19E-G28A-S30T-T38E-T62E-N67D-N71D-K93R-N97L-E111S-Y129M-D139M-P168S-Q184L-G225C-Y235L-K244L-S258D-N261Q-Z298.01Q; N10T-P19E-G28A-S30T-T38E-S59D-N67D-A68S-N71D-K93R-N97D-Y129M-K143Q-P168S-Q184D-G225C-Y235L-K244L-S258D-N261R-T284E-Z298.01Q; P19E-K63L-N71D-Y129M-P168S-Q184L-G225C-K244L; P19E-N67D-N71D-Q78D-K80T-N97D-Y129M-P168S-Q184L-K244L; P19E-T38E-N67D-Y129M-P168S-Q184L-T228V-K244L; P19E-T38E-N67D-Y129M-Q184L-K244L-S258D-N261R; P19E-K63L-N71D-Y129M-P168S-Q184L-K244L-S258D-N261R; P19E-T38E-K63L-N71D-Y129M-P168S-Q184L-K244L-S258D-G259P; K63L-N71D-Y129M-K143R-P168S-Q184L-G225C-T228V-K244L-S258D-G259P oder P19E-T38E-K63L-N71D-Y129M-P168S-Q184L-K244L-S258D-N261R, , wobei die genannte Aminosäurepositionen den Positionen gemäß SEQ ID No. 2 entsprechen.

In einer besonders bevorzugten Ausführungsform umfasst eine solche Mannanasevariante eine Aminosäuresequenz wie sie in einer der SEQ ID NO:13 oder SEQ ID No. 46-91 in WO 2017/079751 A1 wiedergegeben sind. Es wird ausdrücklich und vollinhaltlich auf die Offenbarung der WO 2018/184767 A1 verwiesen.

Eine weitere mögliche bevorzugte Ausführungsform der Erfindung enthält eine Mannanase aus *Bacillus hemicellulosilyticus* oder eine der Varianten davon enthalten sein, die in WO 2018/184767 A1 beschrieben sind. Eine solche Mannanasevariante umfasst eine Aminosäuresequenz, die mindestens 75% (zunehmend bevorzugt mindestens 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% oder 99%) Sequenzidentität zu der Aminosäuresequenz gemäß SEQ ID No. 16 aus WO 2018/184767 A1.

Eine mögliche Ausführungsform der Erfindung kann eine Mannanase aus *Bacillus clausii* oder eine der Varianten davon enthalten, die in WO 2018/184767 A1 beschrieben sind. Eine solche Mannanasevariante umfasst eine Aminosäuresequenz, die mindestens 93% (zunehmend bevorzugt mindestens 94%, 95%, 96%, 97%, 98% oder 99%) Sequenzidentität zu der Aminosäuresequenz gemäß SEQ ID No.12 aus WO 2018/184767 A1 aufweist.

Eine mögliche Ausführungsform der Erfindung kann eine Mannanase aus *Virgibacillus soli* oder eine der Varianten davon enthalten sein, die in WO 2018/184767 A1 beschrieben sind. Eine solche Mannanasevariante umfasst eine Aminosäuresequenz, die mindestens 79% (zunehmend bevorzugt mindestens 80%, 81%, 82%, 83%, 84%, 85%, 86%, 87%, 88%, 89%, 90%, 91%, 92%, 93%, 94%, 95%, 96%, 97%, 98% oder 99%) Sequenzidentität zu der Aminosäuresequenz gemäß SEQ ID No. 20 aus WO 2018/184767 A1 aufweist.

Die erfindungsgemäßen Zusammensetzungen enthalten bevorzugt zusätzlich mindestens einen weiteren Aktivstoff. Aktivstoffe im Sinne der vorliegenden Erfindung sind insbesondere:
- Textilpflegemittel wie Weichmacher, Phobier- und Imprägniermittel gegen Wasser und Wiederanschmutzungen, Bleichaktivatoren, weitere Enzyme, Abtönungsfarbstoffe, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Antistatika, Bügelhilfsmittel, Quell- und Schiebefestmittel, UV-Absorber, kationische Polymere,
- Hautpflegemittel oder
- Parfüm(öl) oder Riechstoffe.

Bevorzugt ist mindestens ein Aktivstoff ausgewählt aus weiteren Enzymen, optischen Aufhellern, Gerüststoffen, Lösemitteln, Antiredepositionsmitteln, Farbtransferinhibitoren, Abtönungsfarbstoff, Konservierungsmitteln, Parfüm oder Mischungen aus mindestens zwei der vorgenannten Aktivstoffe.

Die Zusammensetzung kann ferner ein von der Catecholverbindung gemäß Formel (I) verschiedenes zusätzliches Bleichmittel enthalten. In einer bevorzugten Ausführungsform ist im Wesentlichen kein weiteres Bleichmittel enthalten.

Es ist bevorzugt, wenn die erfindungsgemäße Zusammensetzung zusätzlich mindestens ein weiteres Enzym, insbesondere ausgewählt aus Protease, Lipase, Amylase, Cellulase, Hemicellulase, Tannase, Xylanase, Xanthanase, Xyloglucanase, β-Glucosidase, Pektinase, Carrageenase, Perhydrolase, Oxidase, Oxidoreduktase oder Mischungen daraus (besonders bevorzugt aus Protease, Amylase, Lipase, Cellulase, Pectatlyase oder Mischungen daraus), enthält. Das mindestens eine zusätzliche, weitere Enzym ist von Mannanase verschieden.

Bei der/den Amylase(n) handelt es sich vorzugsweise um eine α-Amylase. Bei der Hemicellulase handelt es sich vorzugsweise um eine β-Glucanase, eine Pektinase, eine Pullulanase. Bei der Cellulase handelt es sich vorzugsweise um ein Cellulase-Gemisch oder eine Einkomponenten-Cellulase, vorzugsweise bzw. überwiegend um eine Endoglucanase und/oder eine Cellobiohydrolase. Bei der Oxidoreduktase handelt es sich vorzugsweise um eine Oxidase, insbesondere eine Cholin-Oxidase, oder um eine Perhydrolase.

Es ist erfindungsgemäß bevorzugt, wenn als weiteres Enzym mindestens eine Protease enthalten ist. Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seiten 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Eine Protease ist ein Enzym, das Peptidbindungen mittels Hydrolyse spaltet. Jedes der Enzyme aus der Klasse E.C. 3.4 fällt erfindungsgemäß darunter (umfassend jede der darunterfallenden dreizehn Unterklassen). Die EC-Nummer entspricht der Enzyme Nomenklatur 1992 der NC-IUBMB, Academic Press, San Diego, California, eingeschlossen der Ergänzungen 1 bis 5, publiziert in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650.

Subtilase benennt eine Untergruppe der Serinproteasen. Die Serinproteasen oder Serinpeptidasen sind eine Untergruppe der Proteasen, die Serin im aktiven Zentrums des Enzyms besitzen, das ein kovalentes Addukt mit dem Substrat bildet. Weiterhin sind die Subtilasen (und die Serineproteasen) dadurch charakterisiert, dass sie neben besagtem Serin mit Histidin und Aspartam zwei weitere Aminosäurereste im aktiven Zentrum aufweisen. Die Subtilasen können in 6 Unterklassen, nämlich die Subtilisin Familie, die Thermitase Familie, die Proteinase K Familie, die Familie der lantibiotischen Peptidasen, die Kexin Familie und die Pyrolysin Familie. Die als Bestandteil der erfindungsgemäßen Zusammensetzungen bevorzugt ausgenommenen oder bevorzugt in reduzierten Mengen enthaltenen Proteasen sind Endopeptidasen (EC 3.4.21).

"Proteaseaktivität" liegt erfindungsgemäß vor, wenn das Enzym proteolytische Aktivität besitzt (EC 3.4). Verschiedenartige Proteaseaktivitäts-Typen sind bekannt: Die drei Haupttypen sind: Trypsin-artig, wobei eine Spaltung des Amidesubstrates nach den Aminosäuren Arg oder Lys bei P1 erfolgt; Chymotrypsin-artig, wobei eine Spaltung nach einer der hydrophoben Aminosäuren bei P1 erfolgt; und Elastase-artig, wobei eine Spaltung des Amidsubstrates nach Ala bei P1 erfolgt.

Die Proteaseaktivität kann nach der in Tenside, Band 7 (1970), S. 125-132 beschriebenen Methode ermittelt werden. Sie wird dementsprechend in PE (Protease-Einheiten) angegeben. Die Proteaseaktivität eines Enzyms lässt sich gemäß gängigen Standardmethoden, wie insbesondere unter Einsatz von BSA als Substrat (Rinderalbumin) und/oder mit der AAPF-Methode.

Überraschenderweise wurde festgestellt, dass eine Protease vom Typ der alkalischen Protease aus Bacillus lentus DSM 5483 oder eine hierzu hinreichend ähnliche Protease (bezogen auf die Sequenzidentität), die mehrere dieser Veränderungen in Kombination aufweist, besonders für den Einsatz in der erfindungsgemäßen Zusammensetzung geeignet und darin vorteilhafterweise verbessert stabilisiert wird. Vorteile des Einsatzes dieser Protease ergeben sich somit insbesondere hinsichtlich der Waschleistung und/oder der Stabilität.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die alkalische Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

In den internationalen Patentanmeldungen WO95/23221A1, WO92/21760A1 und WO2013/060621A1 sind Varianten der alkalischen Protease aus *Bacillus lentus* DSM 5483 offenbart, die für ihren Einsatz in Wasch- oder Reinigungsmitteln geeignet sind. Ferner sind in den internationalen Patentanmeldungen WO2011/032988A1 und WO2016/096714A1 sowie der europäischen Patentanmeldung EP3044302A1 Wasch- und Reinigungsmittel offenbart, die Varianten der alkalischen Protease aus *Bacillus lentus* DSM 5483 enthalten.

Die Konzentration der Protease in der Zusammensetzung beträgt bevorzugt von 0,001-0,1 Gew.-%, weiter vorzugsweise von 0,01 bis 0,06 Gew.-%, bezogen auf aktives Protein.

Die Zusammensetzungen enthalten bevorzugt zusätzlich mindestens eine Cellulase. Eine Cellulase ist ein Enzym. Für Cellulasen können synonyme Begriffe verwendet werden, insbesondere Endoglucanase, Endo-1,4-beta-Glucanase, Carboxymethylcellulase, Endo-1,4-beta-D-Glucanase, beta-1,4-Glucanase, beta-1,4-Endoglucanhydrolase, Celludextrinase oder Avicelase. Entscheidend dafür, ob ein Enzym eine Cellulase im Sinne der Erfindung ist, ist deren Fähigkeit zur Hydrolyse von 1,4-ß-D-glucosidischen Bindungen in Cellulose.

Erfindungsgemäß konfektionierbare Cellulasen (Endoglucanasen, EG) umfassen beispielsweise die pilzliche, Endoglucanase(EG)-reiche Cellulase-Präparation beziehungsweise deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, beziehungsweise der 43 kD-EG aus Humicola insolens DSM 1800. Weitere einsetzbare Handelsprodukte dieses Unternehmens sind Cellusoft^{®}, Renozyme^{®} und Celluclean^{®}. Weiterhin einsetzbar sind beispielsweise Cellulasen, die von dem Unternehmen AB Enzymes, Finnland, unter den Handelsnamen Ecostone^{®} und Biotouch^{®} erhältlich sind, und die zumindest zum Teil auf der 20 kD-EG aus Melanocarpus basieren. Weitere Cellulasen von dem Unternehmen AB Enzymes sind Econase^{®} und Ecopulp^{®}. Weitere geeignete Cellulasen sind aus Bacillus sp. CBS 670.93 und CBS 669.93, wobei die aus Bacillus sp. CBS 670.93 von dem Unternehmen Danisco/Genencor unter dem Handelsnamen Puradax^{®} erhältlich ist. Weitere verwendbare Handelsprodukte des Unternehmens Danisco/Genencor sind "Genencor detergent cellulase L" und IndiAge^{®}Neutra. Auch durch Punktmutationen erhältliche Varianten dieser Enzyme können erfindungsgemäß eingesetzt werden. Besonders bevorzugte Cellulasen sind Thielavia terrestris Cellulasevarianten, die in der internationalen Offenlegungsschrift WO 98/12307 offenbart sind, Cellulasen aus Melanocarpus, insbesondere Melanocarpus albomyces, die in der internationalen Offenlegungsschrift WO 97/14804 offenbart sind, Cellulasen vom EGIII-Typ aus Trichoderma reesei, die in der europäischen Patentanmeldung EP 1 305 432 offenbart sind bzw. hieraus erhältliche Varianten, insbesondere diejenigen, die offenbart sind in den europäischen Patentanmeldungen EP 1240525 und EP 1305432, sowie Cellulasen, die offenbart sind in den internationalen Offenlegungsschriften WO 1992006165, WO 96/29397 und WO 02/099091. Auf deren jeweilige Offenbarung wird daher ausdrücklich verwiesen bzw. deren diesbezüglicher Offenbarungsgehalt wird daher ausdrücklich in die vorliegende Erfindung mit einbezogen.

Erfindungsgemäß besonders bevorzugte Zusammensetzungen sind dadurch gekennzeichnet, dass als zusätzliche Cellulase mindestens eine Cellulase aus *Melanocarpus sp.* oder *Myriococcum sp.* erhältlicher 20K-Cellulase oder solcher, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) dazu aufweist.

Die aus Melanocarpus sp. oder Myriococcum sp. erhältliche 20K-Cellulase ist aus der internationalen Patentanmeldung WO 97/14804 bekannt. Sie besitzt wie dort beschrieben ein Molekulargewicht von etwa 20 kDa und weist bei 50 °C im pH-Bereich von 4 bis 9 mindestens 80% ihrer maximalen Aktivität auf, wobei noch fast 50% der maximalen Aktivität bei pH 10 erhalten bleiben. Sie kann, wie ebenfalls dort beschrieben, aus Melanocarpus albomyces isoliert und in gentechnisch hergestellten Trichoderma reseei-Transformanten produziert werden. Im Sinne der vorliegenden Erfindung brauchbar sind auch Cellulasen, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) zur 20K-Cellulase aufweisen.

K20-Cellulase wird vorzugsweise in solchen Mengen verwendet, dass eine erfindungsgemäße Zusammensetzung eine cellulolytische Aktivität von 1 NCU/g bis 500 NCU/g (bestimmbar durch die Hydrolyse von 1-gewichtsprozentiger Carboxymethylcellulose bei 50 °C und neutralem pH und Bestimmung der dabei freigesetzten reduzierenden Zucker mittels Dinitrosalicylsäure, wie von M.J.Bailey et al. in Enzyme Microb. Technol. 3: 153 (1981) beschrieben; 1 NCU definiert die Enzymmenge, die reduzierenden Zucker in einer Menge erzeugt, die 1 nmol Glukose pro Sekunde entspricht), insbesondere von 2 NCU/g bis 400 NCU/g und besonders bevorzugt von 6 NCU/g bis 200 NCU/g aufweist. Daneben kann die erfindungsgemäße Zusammensetzung gegebenenfalls noch weitere Cellulasen enthalten.

Eine erfindungsgemäße Zusammensetzung enthält vorzugsweise 0,001 mg bis 0,5 mg, insbesondere 0,02 mg bis 0,3 mg an cellulolytischem Protein pro Gramm der gesamten Zusammensetzung. Die Proteinkonzentration kann mit Hilfe bekannter Methoden, zum Beispiel dem Bicinchonsäure-Verfahren (BCA-Verfahren, Pierce Chemical Co., Rockford, IL) oder dem Biuret-Verfahren (A.G. Gornall, C.S. Bardawill und M.M. David, J. Biol. Chem. 177, 751-766, 1948) bestimmt werden.

Es ist erfindungsgemäß wiederum besonders bevorzugt, zusätzlich zu mindestens einer ersten Cellulase aus *Melanocarpus sp.* oder *Myriococcum sp.* erhältlicher 20K-Cellulase oder solcher, die eine Homologie von über 80% (zunehmend bevorzugt von über 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) dazu aufweist mindestens eine weitere von der ersten Cellulase verschiedene zweite Cellulase einzusetzen.

Es ist erfindungsgemäß bevorzugt, wenn die erfindungsgemäßen Zusammensetzungen zusätzlich mindestens eine Lipase enthalten. Erfindungsgemäß bevorzugte Lipase-Enzyme werden ausgewählt aus mindestens einem Enzym der Gruppe, die gebildet wird aus Triacylglycerol-Lipase (E.C. 3.1.1.3) und Lipoprotein-Lipase (E.C. 3.1.1.34) und Monoglycerid-Lipase (E.C. 3.1.1.23).

Ferner ist die in einer erfindungsgemäßen Zusammensetzung bevorzugt enthaltene Lipase natürlicherweise in einem Mikroorganismus der Art *Thermomyces lanuginosus* oder *Rhizopus oryzae* oder *Mucor javanicus* vorhanden oder von vorgenannten natürlicherweise vorhandenen Lipasen per Mutagenese abgeleitet. Besonders bevorzugt enthalten die erfindungsgemäßen Zusammensetzungen mindestens eine Lipase, die natürlicherweise in einem Mikroorganismus der Art *Thermomyces lanuginosus* vorhanden oder sich von vorgenannten natürlicherweise in *Thermomyces lanuginosus* vorhandenen Lipasen per Mutagenese ableitet. Natürlicherweise vorhanden bedeutet in diesem Zusammenhang, dass die Lipase ein eigenes Enzym des Mikroorganismus ist. Die Lipase kann folglich in dem Mikroorganismus von einer Nukleinsäuresequenz exprimiert werden, die Teil der chromosomalen DNA des Mikroorganismus in seiner Wildtyp-Form ist. Sie bzw. die für sie codierende Nukleinsäuresequenz ist folglich in der Wildtyp-Form des Mikroorganismus vorhanden und/oder kann aus der Wildtyp-Form des Mikroorganismus aus diesem isoliert werden. Im Gegensatz hierzu wäre eine nicht natürlicherweise in dem Mikroorganismus vorhandene Lipase bzw. die für sie codierende Nukleinsäuresequenz mit Hilfe gentechnischer Verfahren in den Mikroorganismus gezielt eingebracht worden, so dass der Mikroorganismus um die Lipase bzw. die für sie codierende Nukleinsäuresequenz bereichert worden wäre. Jedoch kann eine Lipase, die natürlicherweise in einem Mikroorganismus der Art *Thermomyces lanuginosus* oder *Rhizopus oryzae* oder *Mucor javanicus* vorhanden ist, aber durchaus rekombinant von einem anderen Organismus hergestellt worden sein.

Der Pilz *Thermomyces lanuginosus* (auch bekannt unter *Humicola lanuginosa*) zählt zur Klasse der Eurotiomycetes (Unterklasse Eurotiomycetidae), hierin zur Ordnung der Eurotiales und hierin zur Familie Trichocomaceae und der Gattung Thermomyces. Der Pilz *Rhizopus oryzae* zählt zur Klasse der Zygomyceten (Unterklasse Incertae sedis), hierin zur Ordnung Mucorales und hierin wiederum zur Familie Mucoraceae und der Gattung Rhizopus. Der Pilz *Mucor javanicus* zählt ebenfalls zur Klasse der Zygomyceten (Unterklasse Incertae sedis), hierin zur Ordnung Mucorales und hierin wiederum zur Familie Mucoraceae, hierin dann zur Gattung Mucor. Die Bezeichnungen *Thermomyces lanuginosus,* Rhizopus oryzae und Mucor javanicus sind die biologischen Artbezeichnungen innerhalb der jeweiligen Gattung.

Erfindungsgemäß bevorzugte Lipasen sind die von dem Unternehmen Amano Pharmaceuticals unter den Bezeichnungen Lipase M-AP10^{®}, Lipase LE^{®} und Lipase F^{®} (auch Lipase JV^{®}) erhältlichen Lipaseenzyme. Die Lipase F^{®} ist beispielsweise natürlicherweise in Rhizopus oryzae vorhanden. Die Lipase M-AP10^{®} ist beispielsweise natürlicherweise in Mucor javanicus vorhanden.

Zusammensetzungen einer ganz besonders bevorzugten Ausführungsform der Erfindung enthalten mindestens eine Lipase, die ausgewählt wird aus mindestens einem oder mehreren Polypeptiden mit einer Aminosäuresequenz, die zu mindestens 90% (und zunehmend bevorzugt zu mindestens 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7%, 99,8%, 99,9%) zur Wildtyp Lipase aus dem Stamm DSM 4109 *Thermomyces lanuginosus* identisch ist. Dabei ist es erneut bevorzugt, wenn ausgehend von besagter Wildtyp Lipase aus dem Stamm DSM 4109 zumindest die Aminosäureänderung N233R vorliegt.

Es sind im Rahmen einer weiteren Ausführungsform insbesondere solche Lipasen abgeleitet von der Wildtyp Lipase aus dem Stamm DSM 4109 erfindungsgemäß bevorzugt verwendbar, die ausgewählt werden aus mindestens einem Lipase-Enzym gemäß mindestens einem der Ansprüche 1 bis 13 der Druckschrift WO 00/60063 A1. Auf die Offenbarung Druckschrift WO 00/60063 A1 wird ausdrücklich vollumfänglich Bezug genommen.

Besonders bevorzugt wird in den Zusammensetzungen der Erfindung mindestens eine Lipase eingesetzt, die abgeleitet ist von der Wildtyp Lipase aus dem Stamm DSM 4109 und in der ausgehend von besagter Wildtyp Lipase mindestens eine Substitution einer elektrisch neutralen oder negativ geladenen Aminosäure durch eine positiv geladene Aminosäure erfolgte. Die Ladung wird in Wasser bei pH 10 bestimmt. Negative Aminosäuren im Sinne der Erfindung sind E, D, Y und C. Positiv geladene Aminosäuren im Sinne der Erfindung sind R, K und H, insbesondere R und K. Neutrale Aminosäure im Sinne der Erfindung sind G, A, V, L, I, P, F, W, S, T, M, N, Q und C, wenn C eine Disulfidbrücke ausbildet.

Im Rahmen dieser Ausführungsform der Erfindung ist es ebenfalls bevorzugt, wenn ausgehend von der Wildtyp Lipase aus dem Stamm DSM 4109 mindestens einen der folgenden Aminosäureaustausche in den Positionen D96L, T213R und/oder N233R, besonders bevorzugt T213R und N233R, vorliegt.

Eine höchst bevorzugte Lipase ist kommerziell unter dem Handelsnamen Lipex^{®} von dem Unternehmen Novozymes (Dänemark) zu beziehen und vorteilhaft in den erfindungsgemäßen Reinigungszusammensetzungen einsetzbar. Besonders bevorzugt ist hierbei die Lipase Lipex^{®} 100 L (ex Novozymes A/S, Dänemark). Bevorzugte Zusammensetzungen sind dadurch gekennzeichnet, dass bezogen auf das Gesamtgewicht der Zusammensetzung besagtes Lipase-Enzym aus Lipex^{®} 100 L in einer Gesamtmenge von 0,01 bis 1,0 Gew.-%, insbesondere von 0,02 bis 0,1 Gew.-%, enthalten ist.

Bevorzugt enthält die erfindungsgemäße Zusammensetzung (besonders bevorzugt neben der bevorzugten Protease vom Typ der alkalischen Protease aus Bacillus lentus DSM 5483 oder neben der hierzu hinreichend ähnlichen Protease (bezogen auf die Sequenzidentität), die mehrere dieser Veränderungen in Kombination aufweist), zusätzlich mindestens eine α-Amylase.

α-Amylasen (E.C. 3.2.1.1) hydrolysieren als Enzym interne α-1,4-glycosidische Bindungen von Stärke und stärkeähnlichen Polymeren. Diese α-Amylase-Aktivität wird beispielsweise den Anmeldungen WO 97/03160 A1 und GB 1296839 zufolge in KNU (Kilo Novo Units) gemessen. Dabei steht 1 KNU für die Enzymmenge, die 5,25 g Stärke (erhältlich von der Fa. Merck, Darmstadt, Deutschland) pro Stunde bei 37°C, pH 5,6 und in Gegenwart von 0,0043 M Calciumionen hydrolysiert. Eine alternative Aktivitäts-Bestimmungsmethode ist die sogenannte DNS-Methode, die beispielsweise in der Anmeldung WO 02/10356 A2 beschrieben wird. Danach werden die durch das Enzym bei der Hydrolyse von Stärke freigesetzten Oligosaccharide, Disaccharide und Glucoseeinheiten durch Oxidation der reduzierenden Enden mit Dinitrosalicysäure (DNS) nachgewiesen. Die Aktivität wird in µmοl reduzierende Zucker (bezogen auf Maltose) pro min und ml erhalten; hierdurch ergeben sich Aktivitätswerte in TAU. Dasselbe Enzym kann über verschiedene Methoden bestimmt werden, wobei die jeweiligen Umrechungsfaktoren je nach Enzym variieren können und somit anhand eines Standards festgelegt werden müssen. Näherungsweise kann man kalkulieren, dass 1 KNU ca. 50 TAU entspricht. Eine weitere Aktivitätsbestimmungsmethode ist die Messung mithilfe des Quick-Start^{®}-Testkits der Fa. Abbott, Abott Park, Illinois, USA.

Ein erfindungsgemäß bevorzugte Einsatzgebiet der erfindungsgemäßen Zusammensetzungen ist die Reinigung von Textilien. Weil Wasch- und Reinigungsmittel für Textilien überwiegend alkalische pH-Werte aufweisen, werden hierfür insbesondere α-Amylasen eingesetzt, die im alkalischen Medium aktiv sind. Solche werden von Mikroorganismen, das heißt Pilzen oder Bakterien, vor allem denen der Gattungen Aspergillus und Bacillus produziert und sekretiert. Ausgehend von diesen natürlichen Enzymen steht weiterhin eine nahezu unüberschaubare Fülle von Varianten zur Verfügung, die über Mutagenese abgeleitet worden sind und je nach Einsatzgebiet spezifische Vorteile aufweisen.

Beispiele hierfür sind die α-Amylasen aus Bacillus licheniformis, aus B. amyloliquefaciens und aus B. stearothermophilus sowie deren für den Einsatz in Wasch- oder Reinigungsmitteln verbesserte Weiterentwicklungen. Das Enzym aus B. licheniformis ist von der Firma Novozymes unter dem Namen Termamyl^{®} und von der Firma Genencor unter dem Namen Purastar^{®}ST erhältlich. Weiterentwicklungsprodukte dieser α-Amylase sind von der Firma Novozymes unter den Handelsnamen Duramyl^{®} und Termamyl^{®}ultra, von der Firma Genencor unter dem Namen Purastar^{®}OxAm und von der Firma Daiwa Seiko Inc., Tokyo, Japan, als Keistase^{®} erhältlich. Die α-Amylase von B. amyloliquefaciens wird von der Firma Novozymes unter dem Namen BAN^{®} vertrieben, und abgeleitete Varianten von der α-Amylase aus B. stearothermophilus unter den Namen BSG^{®} und Novamyl^{®}, ebenfalls von der Firma Novozymes.

Beispiele für α-Amylasen aus anderen Organismen sind die unter den Handelsnamen Fungamyl^{®} von der Firma Novozymes erhältlichen Weiterentwicklungen der α-Amylase aus Aspergillus niger und A. oryzae. Ein weiteres Handelsprodukt ist beispielsweise die Amylase-LT^{®}.

Zum Stand der Technik gehören unter anderem die drei Patentanmeldungen WO 96/23873 A1, WO 00/60060 A2 und WO 01/66712 A2, die von der Fa. Novozymes angemeldet worden sind. WO 96/23873 A1 beschreibt zum Teil mehrere verschiedene Punktmutationen in insgesamt mehr als 30 verschiedenen Positionen in vier verschiedenen Wildtypamylasen und beansprucht solche für alle Amylasen mit mindestens 80% Identität zu einer dieser vier; sie sollen geänderte enzymatische Eigenschaften hinsichtlich der Thermostabilität, der Oxidationsstabilität und der Calciumabhängigkeit aufweisen. Die Anmeldung WO 00/60060 A2 benennt ebenfalls eine Vielzahl an möglichen Aminosäureaustauschen in 10 verschiedenen Positionen an den α-Amylasen aus zwei verschiedenen Mikroorganismen und beansprucht solche für alle Amylasen mit einer Homologie von mindestens 96% Identität zu diesen. WO 01/66712 A2, schließlich, bezeichnet 31 verschiedene, zum Teil mit den zuvor genannten identische Aminosäurepositionen, die in einer der beiden in der Anmeldung WO 00/60060 A2 genannten α-Amylasen mutiert worden sind.

Aus WO 96/23873 A1 geht beispielsweise konkret die Möglichkeit hervor, in den genannten α-Amylasen ein M in Position 9 gemäß der Zählung von AA560 gegen ein L zu ersetzen, in Position 202 M gegen L und die in den Positionen 182 und 183 (beziehungsweise 183 und 184) liegenden Aminosäuren zu deletieren. WO 00/60060 A2 offenbart unter anderem konkret die Aminosäurevariation N195X (das heißt prinzipiell gegen jede andere Aminosäure). WO 01/66712 A2 offenbart unter anderem die Aminosäurevariationen R118K, G186X (darunter insbesondere den hier nicht relevanten Austausch G186R), N299X (darunter insbesondere den hier nicht relevanten Austausch N299A), R320K, E345R und R458K.

Ganz besonders bevorzugt, enthält die erfindungsgemäße Zusammensetzung neben der bevorzugten Protease vom Typ der alkalischen Protease aus Bacillus lentus DSM 5483 oder eine hierzu hinreichend ähnliche Protease (bezogen auf die Sequenzidentität), die mehrere dieser Veränderungen in Kombination aufweist, zusätzlich mindestens eine α-Amylase, die bei Temperaturen zwischen 10 und 20°C eine höhere Aktivität aufweist, als die Amylase mit dem Handelsnamen "Stainzyme 12 L" der Firma Novozymes.

Erfindungsgemäß bevorzugte Zusammensetzungen enthalten α-Amylase in einer Gesamtmenge von 0,01 bis 1,0 Gew.-%, insbesondere von 0,02 bis 0,1 Gew.-%.

Es ist bevorzugt, dass mindestens ein optischer Aufheller enthalten ist, der aus den Substanzklassen der Distyrylbiphenyle, der Stilbene, der 4,4'-Diamino-2,2'-stilbendisulfonsäuren, der Cumarine, der Dihydrochinolinone, der 1,3-Diarylpyrazoline, der Naphthalsäureimide, der Benzoxazol-Systeme, der Benzisoxazol-Systeme, der Benzimidazol-Systeme, der durch Heterocyclen substituierten Pyrenderivate und Mischungen daraus, ausgewählt wird. Diese Substanzklassen an optischen Aufhellern weisen eine hohe Stabilität, eine hohe Licht- und Sauerstoffbeständigkeit und eine hohe Affinität zu Fasern auf.

Besonders gut und stabil lassen sich die folgenden optischen Aufheller, welche aus der Gruppe bestehend aus Dinatrium-4,4'-bis-(2-morpholino-4-anilino-s-triazin-6-ylamino)stilbendisulfonat, Dinatrium-2,2'-bis-(phenyl-styryl)disulfonat, 4,4'-Bis[(4-anilino-6-[bis(2-hydroxyethyl)amino]-1,3,5-triazin-2-yl)amino]stilben-2,2'-disulfonsäure, Hexanatrium-2,2'-[vinylenbis[(3-sulphonato-4,1-phenylen)imino[6-(diethylamino)-1,3,5-triazin-4,2-diyl]imino]]bis-(benzol-1,4-disulfonat), 2,2'-(2,5-Thiophendiyl)bis[5-1,1-dimethylethyl)-benzoxazol (beispielsweise erhältlich als Tinopal^{®} SFP von BASF SE) und/oder 2,5-Bis(benzoxazol-2-yl)thiophen ausgewählt sind, einarbeiten.

Die Zusammensetzung kann erfindungsgemäß weiterhin Gerüststoffe umfassen. Als Gerüststoffe sind beispielsweise polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol.

Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten.

Als Gerüststoffe, die in der erfindungsgemäßen Zusammensetzung enthalten sein können, sind insbesondere auch Silikate, Aluminiumsilikate (insbesondere Zeolithe), Carbonate, Salze organischer Di- und Polycarbonsäuren sowie Mischungen dieser Stoffe zu nennen. Als zusätzliche Gerüststoffe sind insbesondere organische Gerüststoffe geeignet, beispielsweise die in Form ihrer Natriumsalze oder auch als Säuren einsetzbaren Polycarbonsäuren, wobei unter Polycarbonsäuren solche Carbonsäuren verstanden werden, die mehr als eine Säurefunktion tragen. Beispielsweise sind dies Adipinsäure, Bernsteinsäure, Glutarsäure, Äpfelsäure, Weinsäure, Maleinsäure, Fumarsäure, Zuckersäuren, Aminocarbonsäuren, insbesondere Glutaminsäure-N,N-Diessigsäure (GLDA) und Methylglycin-N,N-Diessigsäure (MGDA), sowie Mischungen aus diesen. Als Gerüststoffe sind weiter polymere Polycarboxylate geeignet. Dies sind beispielsweise die Alkalimetallsalze der Polyacrylsäure oder der Polymethacrylsäure, zum Beispiel solche mit einer relativen Molekülmasse von 600 bis 750.000 g/mol. Geeignete Polymere sind insbesondere Polyacrylate, die bevorzugt eine Molekülmasse von 1.000 bis 15.000 g/mol aufweisen. Aufgrund ihrer überlegenen Löslichkeit können aus dieser Gruppe wiederum die kurzkettigen Polyacrylate, die Molmassen von 1.000 bis 10.000 g/mol, und besonders bevorzugt von 1.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Zur Verbesserung der Wasserlöslichkeit können die Polymere auch Allylsulfonsäuren, wie Allyloxybenzolsulfonsäure und Methallylsulfonsäure, als Monomer enthalten. In flüssigen Komponenten werden bevorzugt lösliche Gerüststoffe, wie Acrylpolymere mit einer Molmasse von 1.000 bis 5.000 g/mol eingesetzt.

Besonders bevorzugt werden allerdings lösliche Gerüststoffe, wie beispielsweise Zitronensäure, oder Acryl-polymere mit einer Molmassen von 1.000 bis 5.000 g/mol eingesetzt.

Die Zusammensetzung enthält bevorzugt weiterhin zusätzlich ein oder mehrere nichtwässrige Lösemittel. Geeignete nichtwässrige Lösungsmittel umfassen ein- oder mehrwertige Alkohole oder Glykolether, wie beispielsweise Ethanol, n-Propanol, i-Propanol, Butanolen, Glykol, Propandiol, Butandiol, Methylpropandiol, Glycerin, Glykolen, wie Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, 2,2-dimethyl-4-hydroxymethyl-1,3-dioxolan, Propylen-glykol-t-butylether, Di-n-octylether sowie niedermolekularen Polyalkylenglykolen, wie PEG 400, sowie Mischungen dieser Lösungsmittel.

Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropy-lenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel.

Als weiterer Aktivstoff enthalten die erfindungsgemäßen Zusammensetzungen bevorzugt mindestens einen Abtönungsfarbstoff. Abtönungsfarbstoffe an sich sind dem Fachmann bekannt. In der vorliegenden Erfindung sind alle Abtönungsfarbstoffe geeignet. Bevorzugt werden Abtönungsfarbstoffe eingesetzt, die im Wesentlichen frei von Übergangsmetallen sind.

Insbesondere bevorzugt sind die Abtönungsfarbstoffe Methinblau- und Violetfarbstoffe, Azofarbstoffe, Azinfarbstoffe, Oxazinfarbstoffe, und Xanthenfarbstoffe oder Farbstoffe auf Basis von Anthraquinon, Acridin, Benzodifuran, Benzodifuranon, Carotinoide, Coumarin, Cyanine, Diazahemicyanine, Diphenylmethan, Formazan, Hemicyanide, Indigoide, Methin, Naphthalimide, Naphthoquinone, Nitro- und Nitrosoverbindungen, Oxazine, Pyrazole, Polyethyleniminen, Stilben, Styrol, Triarylmethanen, Triphenylmethanen, Xanthenen, Triphenooxazinen, und Thiophenen oder Mischungen davon.

Geeignete Abtönungsfarbstoffe sind beispielsweise in der WO 2006/004876 A1, WO 2014/089386 A1, WO 2015/110291 A1, WO2015/042209 A1, WO 2012/059363 A1, EP 2852639 A1, EP 2 440645 A1, EP 2 864 464 A1 und WO 2006/055787 A1 offenbart.

In bevorzugten Aspekten ist mindestens ein Abtönungsfarbstoff ausgewählt aus Verbindungen mit der allgemeinen Formel H-1: 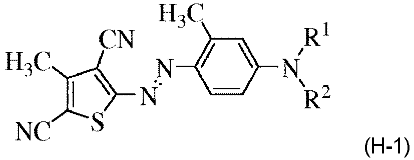 wobei R¹ und R² unabhängig voneinander ausgewählt sind aus [(CH₂CR'HO)ₓ(CH₂CR"HO)_{y}Q], C1-12 Alkyl, C6-10 Aryl, C7-22 Arylalkyl, mit der Maßgabe, dass mindestens einer von R¹ und R² [(CH₂CR'HO)ₓ(CH₂CR"HO)_{y}Q] ist, wobei R` ausgewählt ist aus der Gruppe bestehend aus H, C1-4 Alkyl, CH₂O(CH₂CH₂O)_{z}Q, Phenyl und -CH₂OR⁵; wobei R" ausgewählt ist aus H, C1-4 Alkyl, CH₂O(CH₂CH₂O)_{z}Q, Phenyl und -CH₂OR⁵; wobei 1 oder 2 ≤ x + y ≤ 50, bevorzugt x + y ≤ 25, stärker bevorzugt x + y ≤ 10; wobei y ≥ ; wobei z = o oder 1 bis 20, bevorzugt 0 oder 10 oder 5; und wobei Q ausgewählt ist aus der Gruppe bestehend aus H und Y und wobei Y wie nachstehend definiert ist; mit der Maßgabe, dass der Farbstoff mindestens einen Rest Q der Y ist umfasst; jedes R⁵ ausgewählt ist aus der Gruppe bestehend aus C1-16 linearem oder verzweigtem Alkyl, C6-14 Aryl und C7-16 Arylalkyl; bevorzugt ist R⁵ ausgewählt aus der Gruppe bestehend aus Methyl, Ethyl, Propyl, Isopropyl, Butyl, sec-Butyl, Isobutyl, tert.-Butyl, Hexyl, 2-Ethylhexyl, Octyl, Decyl, Dodecyl, Tetradecyl, Hexadecyl, Phenyl, Benzyl, 2-Phenylethyl, Naphthyl und Mischungen davon; und wobei Y ein organischer Rest ist, der durch Formel H-2 dargestellt wird: 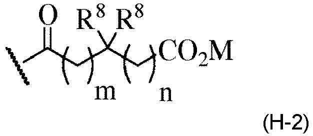 wobei unabhängig voneinander jeder Rest Y, M oder H oder ein ladungsausgleichendes Kation ist; m 0 bis 5, bevorzugt 0, 1, 2 oder 3, ist; n 0 bis 5, bevorzugt 0, 1, 2 oder 3 ist; die Summe von m + n 1 bis 10, bevorzugt 1, 2 oder 3 ist; jedes R⁸ unabhängig ausgewählt aus der Gruppe bestehend aus H und C3-18 oder C4-18 oder sogar C4-7 und/oder C9-18 Alkenyl ist und wobei mindestens ein Rest R⁸ nicht H ist.

Ein bevorzugter Abtönungsfarbstoff auf Antrachinonbasis ist eine Verbindung gemäß Formel (H-3): 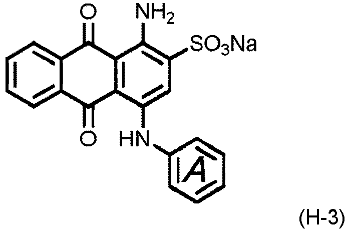 wobei wenn der Ring A substituiert ist durch einen reaktiven Rest, dieser Rest bevorzugt ausgewählt ist aus der Gruppe ausgewählt aus: Dichlortriazinyl, Difluorchlorpyrimidin, Monofluortrazinyl, Monofluorchlortrazinyl, Dichlorchinoxalin, Difluortriazin, Monochlortriazinyl, Trichlorpyrimidin-2-bromprop-2-enamid; 2,3-Dibrompropanamid; und Sulfooxyethylsufonyl. Der Ring A kann dabei zusätzlich durch einen organischen Rest, ausgewählt aus C1-8 Alkyl oder SOsNa substituiert sein.

Ein weiterer bevorzugter Abtönungsfarbstoff auf Antrachinonbasis ist eine Verbindung gemäß Formel (H-4): 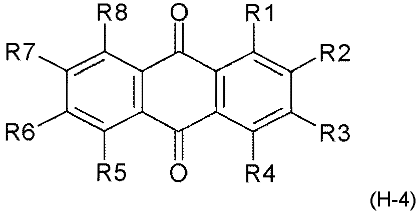 wobei R1, R4, R5 und R8 unabhängig voneinander ausgewählt sind aus den Resten bestehend aus -H, -OH, -NH₂, NHCOCH₃ und -NOz, mit der Maßgabe, dass höchstens ein -Rest ein -NO₂ Rest ist und höchstens zwei -H Reste als R1, R4, R5 und R8 vorliegen; und R2, R3, R6 und R7 ausgewählt sind aus -H, F, Br, Cl oder -NO₂ und -O-Aryl. Besonders bevorzugt sind 1-Hydroxy-4-(p-tolylamino-)antraquinon, 1-Hydroxy-4-[(4-methylphenyl)amino]-9,10-anthracendion und 1-Hydroxy-4-(4-methylanilino)anthrachinon.

Ferner sind Monoazofarbstoffe der Formel (H-5) bevorzugt: 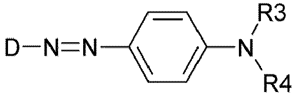 wobei R3 und R4 gegebenenfalls substituierte C2 bis C12 Alkyle sind, die gegebenenfalls eine Ether (-O-) oder Ester Verbrückung in der Kette enthalten, und die Kette gegebenenfalls substituiert ist mit -Cl, -Br, -CN, -NOz, und -SO₂CH₃, und D für einen aromatischen oder heteroaromatischen Rest steht, die gegebenenfalls substituiert sind mit -Cl, -Br, -CN, -NOz, - SO₂CH₃ und -NHCOR, wobei R ausgewählt ist aus -CH₃, -C₂H₅ und -CH₂Cl.

Insbesondere bevorzugt sind Verbindungen der Formeln H-6 und H-7: 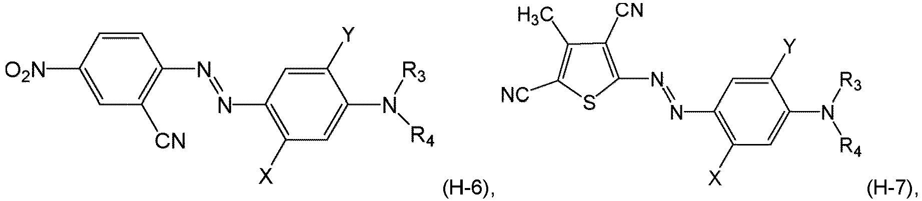 wobei X und Y ausgewählt sind aus -Cl, -Br, -CN, -NOz, -SO₂CH₃ und -NHCOR, wobei R ausgewählt ist aus -CH₃, -C₂H₅ und -CHzCI. Vorzugsweise ist X NHCOCH₃ oder NHCOCH₂Cl.

Weitere bevorzugte Abtönungsfarbstoffe weisen die Formel H-8 auf: 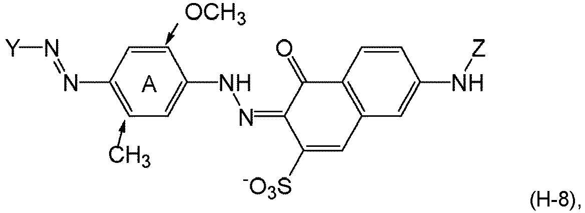 wobei Z H oder Phenyl ist, der Ring A bevorzugt mit einem Methyl oder Methoxyrest an den durch die mit den Pfeilen gekennzeichneten Stellen substituiert ist, wobei der Ring A auch ein Naphthylring sein kann, der Rest Y ein Phenyl- oder Naphthylring ist, welche durch einen Sulphatrest substituiert ist und mono- oder disubstituiert durch zwei Methylreste sein kann.

Auch die Abtönungsfarbstoffe gemäß Formel H-9 sind bevorzugt: 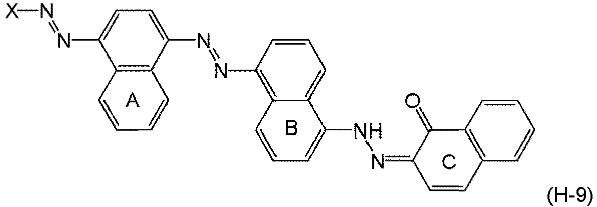 wobei mindestens zwei der Napthylringe A, B und C durch einen Sulphonatrest substituiert sind, der Ring C an der 5-Position durch einen NH₂ oder NHPh-Rest substituiert sein kann, X ein Phenyl- oder Napthylring ist, der mit bis zu 2 Sulphonatresten substituiert ist und der an der 2-Position mit einem -OH Rest substituiert sein kann, und der zudem mit einem NH₂ oder NHPh-Rest substituiert sein kann.

Ferner kann mindestens ein Abtönungsfarbstoff folgende Formel H-10 aufweisen: 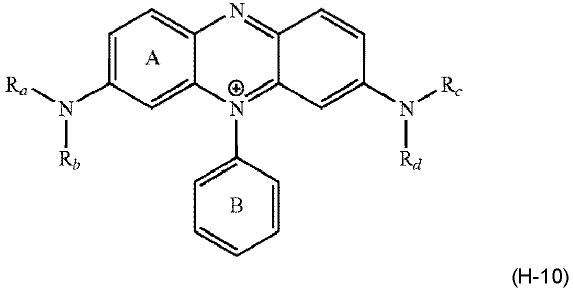 wobei Rₐ, R_{b}, R_{c} und R_{d} ausgewählt sind aus: H, einem verzweigten oder linearem C1-C7 Alkylrest, Benzyl, Phenyl, und Naphthyl, wobei der Farbstoff mit mindestens einem Rest -SO₃⁻ oder -COO⁻, bevorzugt zwei -SO₃⁻ Resten, substituiert ist; der Ring B keine negativ geladenen Reste oder Salze davon aufweist; und der Ring A so substituiert sein kann, dass er Naphthyl bildet; wobei der Farbstoff gegebenenfalls ferner substituiert sein kann durch Reste ausgewählt aus: Amin, Methyl, Ethyl, Hydroxyl, Methoxy, Ethoxy, Phenoxy, CI, Br, I, F und NOz.

Weitere bevorzugte Abtönungsfarbstoffe weisen die Formel H-11 auf: 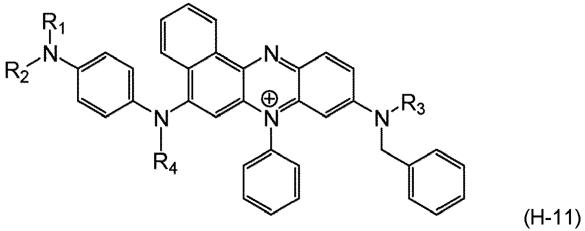 wobei, R1, R2, R3 und R4 ausgewählt sind aus der Gruppe bestehend aus: H, Me, Et, n-Pr und i-Pr; und der Farbstoff gegebenenfalls durch einen Methoxyrest substituiert ist.

Weitere bevorzugte Abtönungsfarbstoffe weisen die Formel H-12 auf: 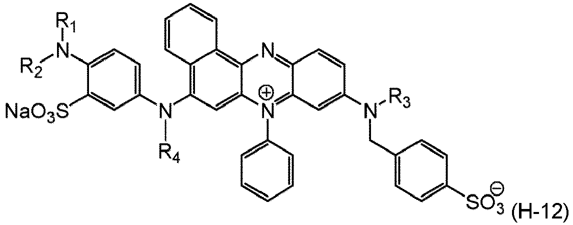 wobei R1, R2, R3 und R4 ausgewählt sind aus der Gruppe bestehend aus: H, Me, Et, n-Pr und i-Pr; und der Farbstoff gegebenenfalls durch einen Methoxyrest substituiert ist.

Ferner sind Abtönungsfarbstoffe mit der Formel H-13 bevorzugt: 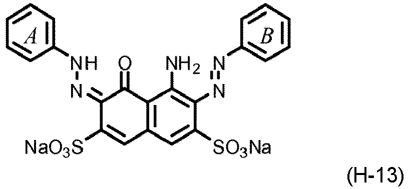 wobei einer oder beide Ringe A und B mit einem reaktiven Rest substituiert sind, bevorzugt mit - SOsNa, und gegebenenfalls ferner -CH₃, -C₂H₅ und -OCH₃.

Weitere bevorzugte Abtönungsfarbstoffe weisen die Formel H-14 auf: 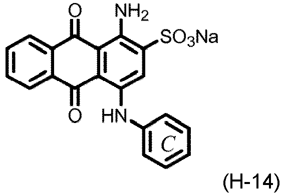 wobei Ring C mit einem reaktiven Rest substituiert sind, bevorzugt mit -SOsNa, und gegebenenfalls ferner -CH₃, -C₂H₅ und -OCH₃.

Ebenfalls bevorzugte Abtönungsfarbstoffe weisen die Formel H-15 auf: 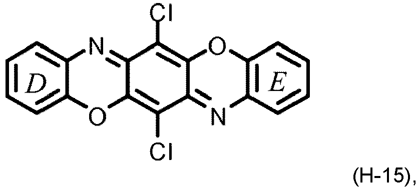 wobei Ringe D und E mit einem reaktiven Rest substituiert sind, bevorzugt mit -SOsNa.

Beispielhafte bevorzugte Verbindungen für einen erfindungsgemäß einsetzbaren Abtönungsfarbstoff sind 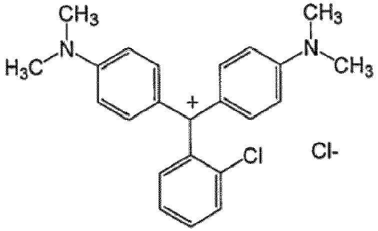 auch bekannt als Basic Blue 1, 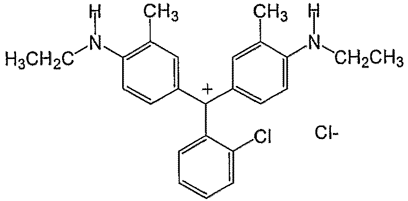 auch bekannt als Basic Blue 5, 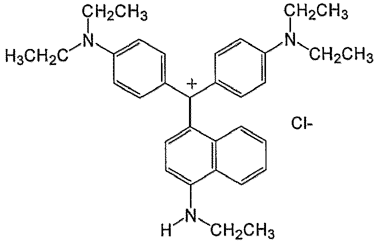 auch bekannt als Basic Blue 7, 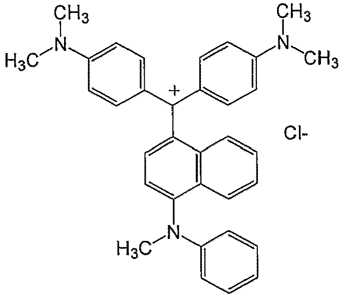 auch bekannt als Basic Blue 8, 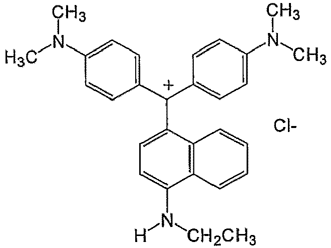 auch bekannt als Basic Blue 11, 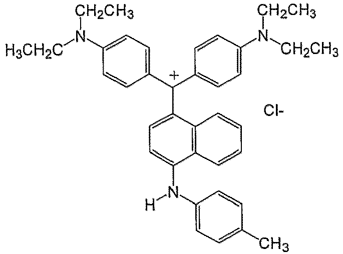 auch bekannt als Basic Blue 15, 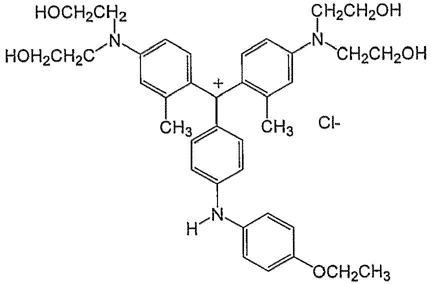 auch bekannt als Basic Blue 18, 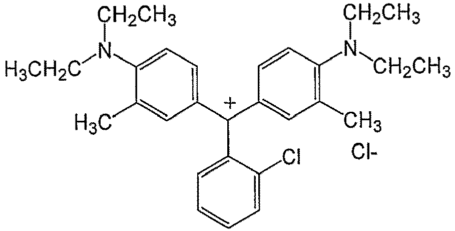 auch bekannt als Basic Blue 23, 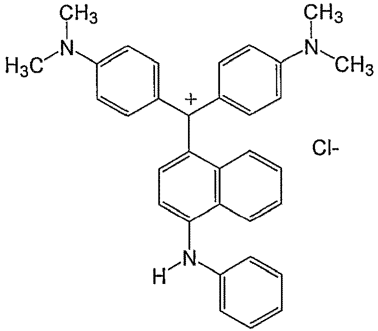 auch bekannt als Basic Blue 26, 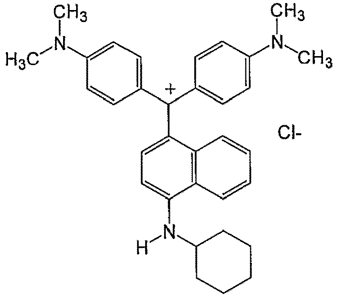 auch bekannt als Basic Blue 55, 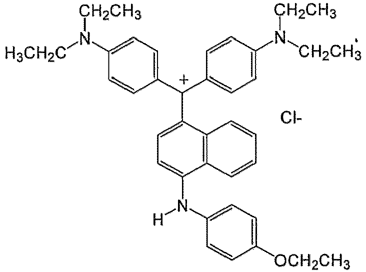 auch bekannt als Basic Blue 81, 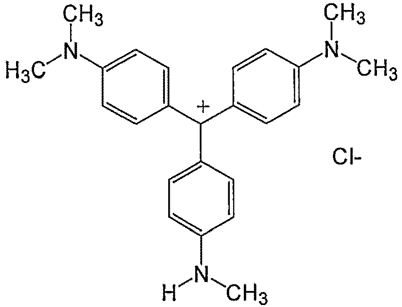 auch bekannt als Basic Violet 1, 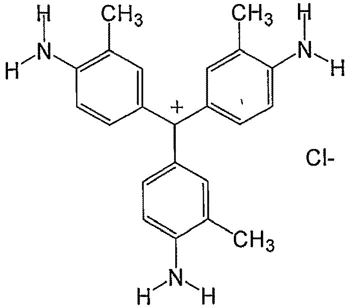 auch bekannt als Basic Violet 2, 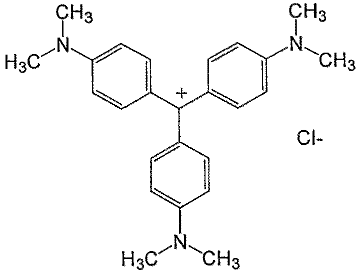 auch bekannt als Basic Violet 3, 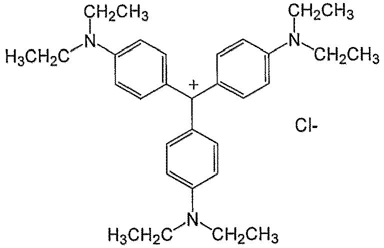 auch bekannt als Basic Violet 4, 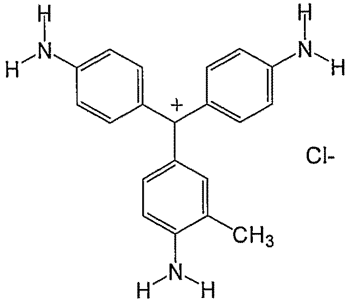 auch bekannt als Basic Violet 14, 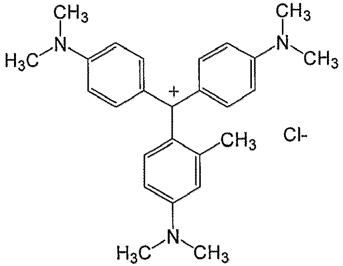 auch bekannt als Basic Violet 23, 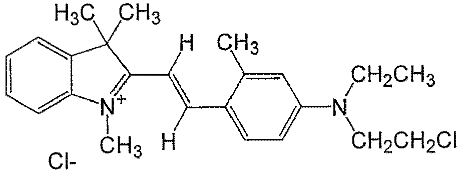 auch bekannt als Basic Violet 7, 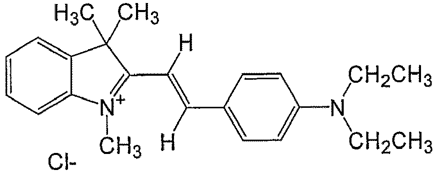 auch bekannt als Basic Violet 16, 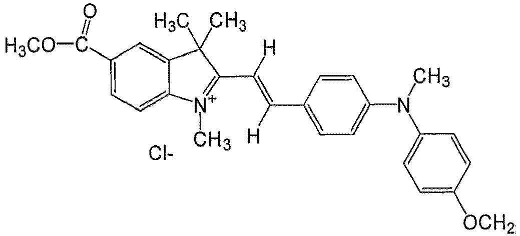 auch bekannt als Basic Violet 21, 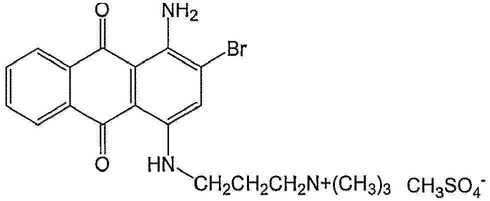 auch bekannt als Basic Blue 21, 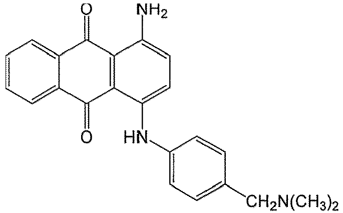 auch bekannt als Basic Blue 47, 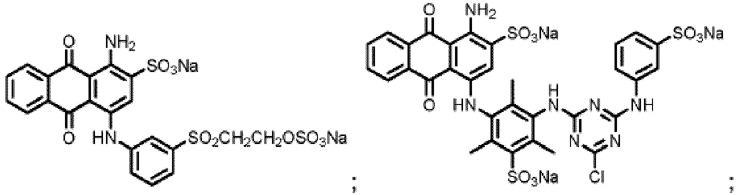 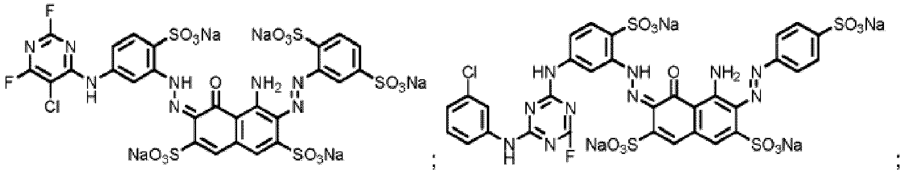 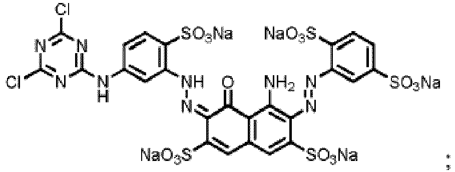 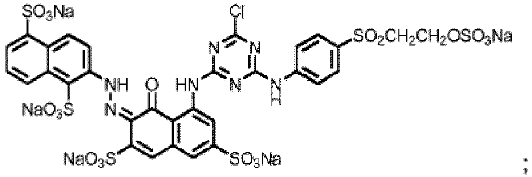 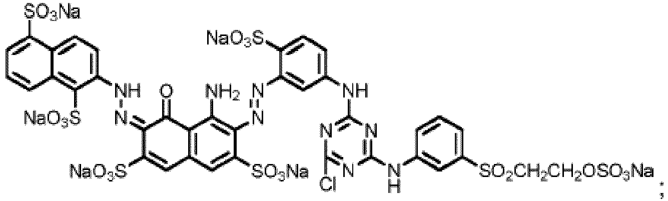 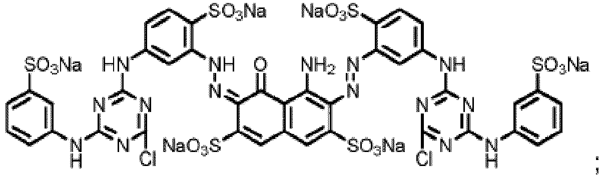 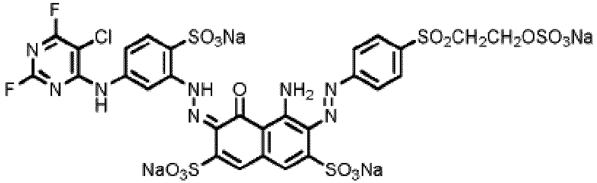 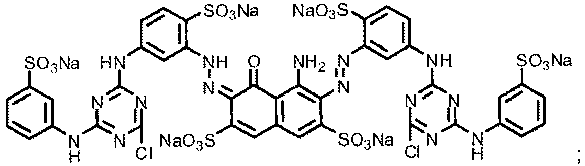 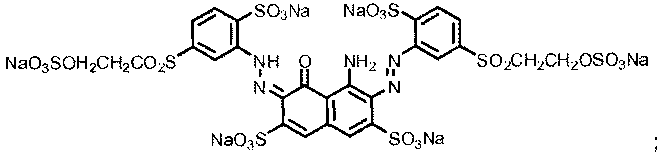 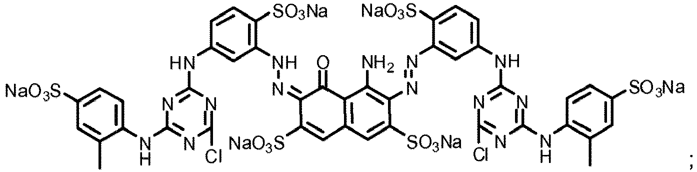 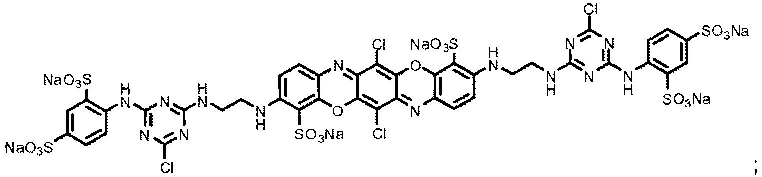 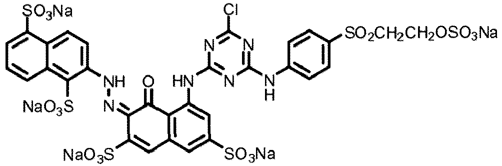 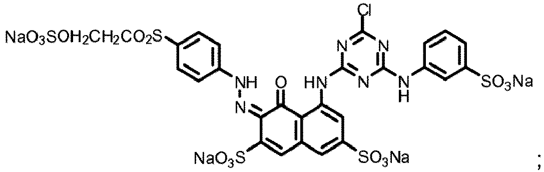 and 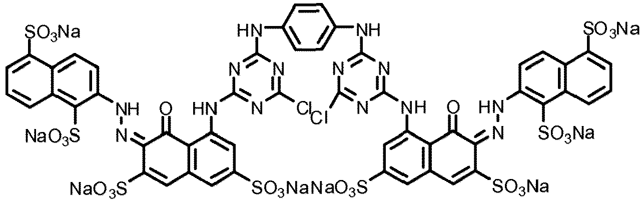 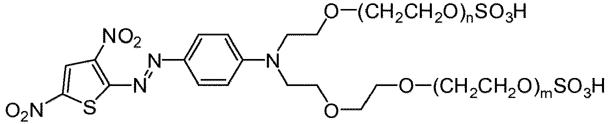 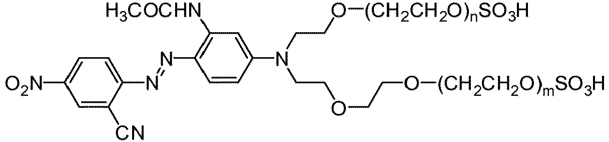 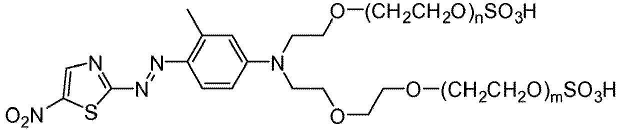 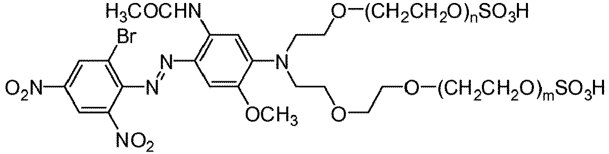 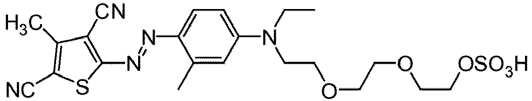 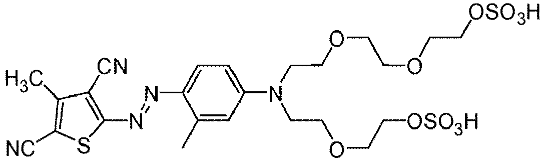 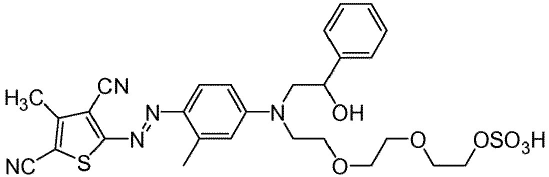 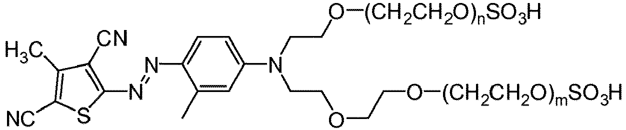 wobei in den vorstehenden fünf Verbindungen n eine ganze Zahl von 0 bis 7 ist und m eine ganze Zahl von 0 bis 7 ist.

Zusätzlich können die erfindungsgemäßen Zusammensetzungen weiterhin auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Mittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen. Solche sind beispielsweise unter dem Handelsnamen Texcare^{®} kommerziell erhältlich.

Als Antiredepositionsmittel sind insbesondere auf (Co)Polymere auf Basis von Polyethylenimin, Polyvinylacetat und Polyethylenglykol einsetzbar, bevorzugt in Mischungen mit Antiredepositionsmitteln.

Die Zusammensetzung kann bevorzugt auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Zusammensetzung, eingesetzt werden. Es ist bevorzugt, dass der Farbübertragungsinhibitor ein Polymer oder Copolymer von cyclischen Aminen wie beispielsweise Vinylpyrrolidon und/oder Vinylimidazol ist. Als Farbübertragungsinhibitor geeignete Polymere umfassen Polyvinylpyrrolidon (PVP), Polyvinylimidazol (PVI), Copolymere von Vinylpyrrolidon und Vinylimidazol (PVP/PVI), Polyvinylpyridin-N-oxid, Poly-N-carboxymethyl-4-vinylpyridiumchlorid, Polyethylenglycolmodifizierte Copolymere von Vinylpyrrolidon und Vinylimidazol sowie Mischungen daraus. Besonders bevorzugt werden Polyvinylpyrrolidon (PVP), Polyvinylimidazol (PVI) oder Copolymere von Vinylpyrrolidon und Vinylimidazol (PVP/PVI) als Farbübertragungsinhibitor eingesetzt. Die eingesetzten Polyvinylpyrrolidone (PVP) besitzen bevorzugt ein mittleres Molekular gewicht von 2.500 bis 400.000 und sind kommerziell von ISP Chemicals als PVP K 15, PVP K 30, PVP K 60 oder PVP K 90 oder von der BASF als Sokalan^{®} HP 50 oder Sokalan^{®} HP 53 erhältlich. Die eingesetzten Copolymere von Vinylpyrrolidon und Vinylimidazol (PVP/PVI) weisen vorzugsweise ein Molekulargewicht im Bereich von 5.000 bis 100.000 g/mol auf. Kommerziell erhältlich ist ein PVP/PVI-Copolymer beispielsweise von der BASF unter der Bezeichnung Sokalan^{®} HP 56. Ein weiterer äußerst bevorzugt einsetzbarer Farbübertragungsinhibitor sind Polyethylenglycolmodifizierte Copolymere von Vinylpyrrolidon und Vinylimidazol, welche beispielsweise unter der Bezeichnung Sokalan^{®} HP 66 von der BASF erhältlich sind.

Die Zusammensetzung wird insbesondere im Rahmen eines Waschprozesses für Textilien eingesetzt. Die hierin beschriebenen Zusammensetzungen eignen sich einerseits als Waschhilfsmittel, die als Textilvor- und Nachbehandlungsmittel bei der Textilwäsche verwendet werden, also als solche Mittel, mit denen das Wäschestück vor der eigentlichen Wäsche in Kontakt gebracht wird, beispielsweise zum Anlösen hartnäckiger Verschmutzungen.

Die Zusammensetzung kann in einer für einen Waschgang vorgefertigten Portion (z.B. als wasserlöslichen Mehrkammerpouch) oder ein Mehrkammer-Vorratsgebinde mit eventueller Mischkammer vorliegen.

Eine tensidhaltige Flotte ist im Sinne der Erfindung eine durch Einsatz des Mehrkomponenten-Waschmittels der vorliegenden Erfindung unter Verdünnung mit mindestens einem Lösemittel (bevorzugt Wasser) erhältliche, flüssige Zubereitung für die Behandlung eines Substrats. Als Substrat kommen Gewebe oder Textilien (wie z.B. Kleidung) in Frage. Bevorzugt werden die erfindungsgemäßen Zusammensetzungen zur Bereitstellung einer tensidhaltigen Flotte im Rahmen maschineller Reinigungsverfahren verwendet, wie sie z.B. von einer Waschmaschine für Textilien ausgeführt werden.

Handelt es sich um ein Mehrkomponenten-Waschmittel, so können die weiteren Komponenten des Mehrkomponenten-Waschmittels in jeder nach dem Stand der Technik etablierten und/oder jeder zweckmäßigen Darreichungsform vorliegen. Dazu zählen beispielsweise flüssige, gelförmige oder pastöse Darreichungsformen. Der Behälter ist bevorzugt ein Pouch mit zwei, drei, vier, fünf, sechs, sieben oder acht Kammern, sowohl in Großgebinden als auch portionsweise abgepackt.

Es ist aber erfindungsgemäß ganz besonders bevorzugt, wenn das Mehrkomponenten-Waschmittel ausschließlich flüssige Komponenten umfasst, wobei die flüssigen Komponenten bevorzugt von einer Umhüllung getrennt voneinander konfektioniert sind.

Bei dem Mehrkomponenten-Waschmittel handelt es sich in einer weiteren besonders bevorzugten Ausführungsform um ein Mehrkomponenten-Color-Waschmittel, insbesondere ein flüssiges, also ein Textilwaschmittel für gefärbte Textilien.

Die erfindungsgemäßen Waschmittel enthaltend bevorzugt mindestens ein Alkalisierungsmittel oder dessen Salz in einer Gesamtmenge von 1 bis 20 Gew.-%, bevorzugt von 2 bis 15 Gew.-%.

Die Gesamtmenge des Alkalisierungsmittels und dessen Salz, bzw die Gesamtmenge aller folgenden bevorzugten Vertreter, wird auf Basis der Basenform berechnet, d.h. wenn das Alkalisierungsmittel im erfindungsgemäßen Waschmittel (teilweise) in seiner Salzform vorliegt, wird bei der Mengenberechnung das Gegenion vernachlässigt und für den Salzanteil nur die Basenform ohne das aufgenommene Proton angenommen.

Die Alkalisierungsmittel werden bevorzugt ausgewählt aus (C₂ bis C₆)-Alkanolamin, Natriumhydroxid, Kaliumhydroxid, Natriumcarbonat, Natriumhydrogencarbonat oder Mischungen daraus.

Unter dem Begriff (C₂ bis C₆)-Alkanolamin sind erfindungsgemäß organische Aminverbindungen zu verstehen, die ein Kohlenstoffgerüst aus zwei bis sechs Kohlenstoffatomen besitzen, an das mindestens eine Aminogruppe (bevorzugt genau eine Aminogruppe) und mindestens eine Hydroxygruppe (wiederum bevorzugt genau eine Hydroxygruppe) bindet.

Bei erfindungsgemäß bevorzugten (C₂ bis C₆)-Alkanolaminen handelt es sich um primäre Amine.

Im Rahmen der Erfindung ist es bevorzugt mindestens ein (C₂ bis C₆)-Alkanolamin mit genau einer Aminogruppe einzusetzen. Hierbei handelt es sich wiederum bevorzugt um ein primäres Amin.

Die erfindungsgemäße Zusammensetzung enthält bevorzugt mindestens ein (C₂ bis C₆)-Alkanolamin ausgewählt unter 2-Aminoethan-1-ol (Monoethanolamin), Tris(2-hydroxyethyl)amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol (insbesondere unter 2-Aminoethan-1-ol, 2-Amino-2-methylpropan-1-ol, 2-Amino-2-methyl-propan-1,3-diol), oder Mischungen davon. Monoethanolamin hat sich als ganz besonders geeignetes (C₂ bis C₆)-Alkanolamin als Alkalisierungsmittel erwiesen.

(C₂ bis C₆)-Alkanolamin oder dessen Salz ist besonders bevorzugt in einer Gesamtmenge von 1 bis 20 Gew.-%, bevorzugt von 2 bis 15 Gew.-%, in den erfindungsgemäßen Waschmitteln enthalten, jeweils bezogen auf die basische Form.

Erfindungsgemäß befindet sich die flüssige Zusammensetzung bevorzugt in einem Behälter (Pouch) aus wasserlöslichem Material. Der Behälter für das besagte Mehrkomponenten-Waschmittel umfasst mindestens zwei räumlich voneinander getrennte Kammern (Mehrkammerpouch), beispielsweise 2, 3, 4, 5, 6, 7 oder 8 Kammern. Diese Kammern sind derart voneinander getrennt, dass die enthaltenen flüssigen oder flüssigen und festen Komponenten des Waschmittels nicht miteinander in Kontakt treten. Diese Trennung kann beispielsweise durch eine Wand erfolgen, die aus demselben Material besteht wie der Behälter selbst.

Es ist daher erfindungsgemäß besonders bevorzugt, das Mehrkomponenten-Waschmittel, in einem Behälter aus wasserlöslichem Material mit mindestens zwei voneinander getrennten Kammern zu konfektionieren.

Wasserlöslich im Sinne der vorliegenden Erfindung ist ein Material, wenn sich bei 20°C 0,1 g des Materials unter Rühren (Rührgeschwindigkeit Magnetrührer 300 rpm, Rührstab: 6,8 cm lang, Durchmesser 10 mm, Becherglas 1000mL niedrige Form der Fa. Schott, Mainz) innerhalb von 600 Sekunden derart in 800 mL Wasser auflöst, dass mit dem bloßen Auge keine einzelnen festförmigen Partikel des Materials mehr sichtbar sind.

Die Wasserlöslichkeit des für die Herstellung von Pouches zur Umhüllung genutzten Materials in Form eines Films kann mit Hilfe eines in einem quadratischen Rahmen (Kantenlänge auf der Innenseite: 20 mm) fixierten quadratischen Films des besagten Materials (Film: 22 x 22 mm mit einer Dicke von 76 µm) nach folgendem Messprotokoll bestimmt werden. Besagter gerahmter Film wird in 800 mL auf 20 °C temperiertes, destilliertes Wasser in einem 1 Liter Becherglas mit kreisförmiger Bodenfläche (Fa. Schott, Mainz, Becherglas 1000 mL, niedrige Form) eingetaucht, so dass die Fläche des eingespannten Films im rechten Winkel zur Bodenfläche des Becherglases angeordnet ist, die Oberkante des Rahmens 1 cm unter der Wasseroberfläche ist und die Unterkante des Rahmens parallel zur Bodenfläche des Becherglases derart ausgerichtet ist, dass die Unterkante des Rahmens entlang des Radius der Bodenfläche des Becherglases verläuft und die Mitte der Unterkante des Rahmens über der Mitte des Radius des Becherglasbodens angeordnet ist. Das Material sollte sich unter Rühren (Rührgeschwindigkeit Magnetrührer 300 rpm, Rührstab: 6,8 cm lang, Durchmesser 10 mm) innerhalb von 600 Sekunden derart auflösen, dass mit dem bloßen Auge keine einzelnen festförmigen Folienpartikel mehr sichtbar sind.

Das wasserlösliche oder wasserdispergierbare Material kann ein Polymer, ein Copolymer oder Mischungen dieser umfassen. Wasserlösliche Polymere im Sinne der Erfindung sind solche Polymere, die bei Raumtemperatur in Wasser zu mehr als 2,5 Gew.-% löslich sind.

Bevorzugte wasserlösliche Materialien umfassen vorzugsweise mindestens anteilsweise wenigstens eine Substanz aus der Gruppe bestehend aus (acetalisierter) Polyvinylalkohol, Polyvinylpyrrolidon, Polyethylenoxid, Gelatine, mit Sulphat, Carbonat und/oder Citrat substituierte Polyvinylalkohole, Polyalkylenoxide, Acrylamide, Celluloseester, Celluloseether, Celluloseamide, Cellulose, Polyvinylacetate, Polycarbonsäuren und deren Salze, Polyaminosäuren oder Peptide, Polyamide, Polyacrylamide, Copolymere von Maleinsäure und Acrylsäure, Copolymere von Acrylamiden und (Meth)Acrylsäure, Polysaccaride, wie beispielsweise Stärke oder Guar-Derivate, Gelatine und unter den INCI Bezeichnungn Polyquaternium 2, Polyquaternium 17, Polyquaternium 18 und Polyquaternium 27 auf. Besonders bevorzugt ist das wasserlösliche Material ein Polivinylalkohol.

In einer Ausführungsform der Erfindung umfasst das wasserlösliche Material Mischungen unterschiedlicher Substanzen. Solche Mischungen ermöglichen die Einstellung der mechanischen Eigenschaften des Behälters und können den Grad der Wasserlöslichkeit beeinflussen.

Das wasserlösliche Material enthält bevorzugt mindestens einen Polyvinylalkohol und/oder mindestens ein Polyvinylalkoholcopolymer. "Polyvinylalkohol" (Kurzzeichen PVAL oder PVA gelegentlich auch PVOH) ist dabei die Bezeichnung für Polymere der allgemeinen Struktur 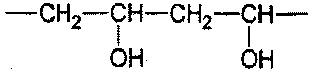 die in geringen Anteilen (ca. 2%) auch Struktureinheiten des Typs 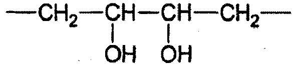 enthalten.

Handelsübliche Polyvinylalkohole, die als weiß-gelbliche Pulver oder Granulate mit Polymerisationsgraden im Bereich von ca. 100 bis 2500 (Molmassen von ca. 4000 bis 100.000 g/mol) angeboten werden, haben Hydrolysegrade von 98 bis 99 Mol-% beziehungsweise 87 bis 89 Mol-%, enthalten also noch einen Restgehalt an Acetyl-Gruppen. Charakterisiert werden die Polyvinylalkohole von Seiten der Hersteller durch Angabe des Polymerisationsgrades des Ausgangspolymeren, des Hydrolysegrades, der Verseifungszahl beziehungsweise der Lösungsviskosität.

Polyvinylalkohole sind abhängig vom Hydrolysegrad löslich in Wasser und wenigen stark polaren organischen Lösungsmitteln (Formamid, Dimethylformamid, Dimethylsulfoxid); von (chlorierten) Kohlenwasserstoffen, Estern, Fetten und Ölen werden sie nicht angegriffen. Polyvinylalkohole werden als toxikologisch unbedenklich eingestuft und sind biologisch zumindest teilweise abbaubar. Die Wasserlöslichkeit kann man durch Nachbehandlung mit Aldehyden (Acetalisierung), durch Komplexierung mit Ni- oder Cu-Salzen oder durch Behandlung mit Dichromaten, Borsäure od. Borax verringern. Die Beschichtungen aus Polyvinylalkohol sind weitgehend undurchdringlich für Gase wie Sauerstoff, Stickstoff, Helium, Wasserstoff, Kohlendioxid, lassen jedoch Wasserdampf hindurchtreten.

Im Rahmen der vorliegenden Erfindung ist es bevorzugt, dass das wasserlösliche Material wenigstens anteilsweise einen Polyvinylalkohol umfasst, dessen Hydrolysegrad 70 bis 100 Mol-%, vorzugsweise 80 bis 90 Mol-%, besonders bevorzugt 81 bis 89 Mol-% und insbesondere 82 bis 88 Mol-% beträgt. In einer bevorzugten Ausführungsform besteht das wasserlösliche Material zu mindestens 20 Gew.-%, besonders bevorzugt zu mindestens 40 Gew.-%, ganz besonders bevorzugt zu mindestens 60 Gew.-% und insbesondere zu mindestens 80 Gew.-% aus einem Polyvinylalkohol, dessen Hydrolysegrad 70 bis 100 Mol-%, vorzugsweise 80 bis 90 Mol%, besonders bevorzugt 81 bis 89 Mol-% und insbesondere 82 bis 88 Mol% beträgt.

Die vorstehend beschriebenen Polyvinylalkohole sind kommerziell breit verfügbar, beispielsweise unter dem Warenzeichen Mowiol^{®} (Clariant). Im Rahmen der vorliegenden Erfindung besonders geeignete Polyvinylalkohole sind beispielsweise Mowiole 3-83, Mowiol^{®} 4-88, Mowiol^{®} 5-88, Mowiol^{®} 8-88 sowie L648, L734, Mowiflex LPTC 221 ex KSE sowie die Compounds der Firma Texas Polymers wie beispielsweise Vinex 2034.

Bevorzugte Polyvinylalkoholcopolymere umfassen neben Vinylalkohol Dicarbonsäuren als weitere Monomere. Geeignete Dicarbonsäure sind Itaconsäure, Malonsäure, Bernsteinsäure und Mischungen daraus, wobei Itaconsäure bevorzugt ist.

Ebenso bevorzugte Polyvinylalkoholcopolymere umfassen neben Vinylalkohol eine ethylenisch ungesättige Carbonsäure, deren Salz oder deren Ester. Besonders bevorzugt enthalten solche Polyvinylalkoholcopolymere neben Vinylalkohol Acrylsäure, Methacrylsäure, Acrylsäureester, Methacrylsäureester oder Mischungen daraus.

Die Wasserlöslichkeit von Polyvinylalkoholpolymer kann durch Nachbehandlung mit Aldehyden (Acetalisierung) oder Ketonen (Ketalisierung) verändert werden. Als besonders bevorzugt und aufgrund ihrer ausgesprochen guten Kaltwasserlöslichkeit besonders vorteilhaft haben sich hierbei Polyvinylalkohole herausgestellt, die mit den Aldehyd beziehungsweise Ketogruppen von Sacchariden oder Polysacchariden oder Mischungen hiervon acetalisiert beziehungsweise ketalisiert werden.

Weiterhin lässt sich die Wasserlöslichkeit durch Komplexierung mit Ni- oder Cu-Salzen oder durch Behandlung mit Dichromaten, Borsäure, Borax verändern und so gezielt auf gewünschte Werte einstellen. Bevorzugt sind diese nicht enthalten. Folien aus PVAL sind weitgehend undurchdringlich für Gase wie Sauerstoff, Stickstoff, Helium, Wasserstoff, Kohlendioxid, lassen jedoch Wasserdampf hindurchtreten.

Dem als wasserlösliches Material geeigneten Folienmaterial kann neben Polyvinylalkohol zusätzlich Polymere, ausgewählt aus der Gruppe umfassend Acrylsäure-haltige Polymere, Polyacrylamide, Oxazolin-Polymere, Polystyrolsulfonate, Polyurethane, Polyester, Polyether Polymilchsäure, und/oder Mischungen der vorstehenden Polymere, zugesetzt sein.

Geeignete wasserlösliche Folien zum Einsatz als wasserlösliches Material der wasserlöslichen Portion gemäß der Erfindung sind Folien, die unter der Bezeichnung Monosol M8630 oder M8720 von MonoSol LLC vertrieben werden. Andere geeignete Folien umfassen Folien mit der Bezeichnung Solublon^{®} PT, Solublon^{®} KA, Solublon^{®} KC oder Solublon^{®} KL von der Aicello Chemical Europe GmbH oder die Folien VF-HP von Kuraray.

Bevorzugte wasserlösliche Materialien sind dadurch gekennzeichnet, dass sie Hydroxypropylmethylcellulose (HPMC) umfassen, die einen Substitutionsgrad (durchschnittliche Anzahl von Methoxygruppen pro Anhydroglucose-Einheit der Cellulose) von 1,0 bis 2,0, vorzugsweise von 1,4 bis 1,9, und eine molare Substitution (durchschnittliche Anzahl von Hydroxypropoxylgruppen pro Anhydroglucose-Einheit der Cellulose) von 0,1 bis 0,3, vorzugsweise von 0,15 bis 0,25, aufweist.

Polyvinylpyrrolidone, kurz als PVP bezeichnet, werden durch radikalische Polymerisation von 1-Vinylpyrrolidon hergestellt. Handelsübliche PVP haben Molmassen im Bereich von ca. 2.500 bis 750.000 g/mol und werden als weiße, hygroskopische Pulver oder als wässrige Lösungen angeboten.

Polyethylenoxide, kurz PEOX, sind Polyalkylenglykole der allgemeinen Formel

H-[O-CH₂-CH₂]ₙ-OH

die technisch durch basisch katalysierte Polyaddition von Ethylenoxid (Oxiran) in meist geringe Mengen Wasser enthaltenden Systemen mit Ethylenglykol als Startmolekül hergestellt werden. Sie haben üblicherweise Molmassen im Bereich von ca. 200 bis 5.000.000 g/mol, entsprechend Polymerisationsgraden n von ca. 5 bis >100.000. Polyethylenoxide besitzen eine äußerst niedrige Konzentration an reaktiven Hydroxy-Endgruppen und zeigen nur noch schwache Glykol-Eigenschaften.

Gelatine ist ein Polypeptid (Molmasse: ca. 15.000 bis >250.000 g/mol), das vornehmlich durch Hydrolyse des in Haut und Knochen von Tieren enthaltenen Kollagens unter sauren oder alkalischen Bedingungen gewonnen wird. Die Aminosäuren-Zusammensetzung der Gelatine entspricht weitgehend der des Kollagens, aus dem sie gewonnen wurde, und variiert in Abhängigkeit von dessen Provenienz. Die Verwendung von Gelatine als wasserlösliches Hüllmaterial ist insbesondere in der Pharmazie in Form von Hart- oder Weichgelatinekapseln äußerst weit verbreitet. In Form von Folien findet Gelatine wegen ihres im Vergleich zu den vorstehend genannten Polymeren hohen Preises nur geringe Verwendung.

Bevorzugt sind im Rahmen der vorliegenden Erfindung wasserlösliche Materialien, welche ein Polymer aus der Gruppe Stärke und Stärkederivate, Cellulose und Cellulosederivate, insbesondere Methylcellulose und Mischungen hieraus umfassen.

Stärke ist ein Homoglykan, wobei die Glucose-Einheiten α-glykosidisch verknüpft sind. Stärke ist aus zwei Komponenten unterschiedlichen Molekulargewichts (MG) aufgebaut: aus ca. 20 bis 30% geradkettiger Amylose (MG ca. 50.000 bis 150.000 g/mol) und 70 bis 80% verzweigtkettigem Amylopektin (MG ca. 300.000 bis 2.000.000 g/mol). Daneben sind noch geringe Mengen Lipide, Phosphorsäure und Kationen enthalten. Während die Amylose infolge der Bindung in 1,4-Stellung lange, schraubenförmige, verschlungene Ketten mit etwa 300 bis 1.200 Glucose-Molekülen bildet, verzweigt sich die Kette beim Amylopektin nach durchschnittlich 25 Glucose-Bausteinen durch 1,6-Bindung zu einem astähnlichen Gebilde mit etwa 1.500 bis 12.000 Molekülen Glucose. Neben reiner Stärke sind zur Herstellung wasserlöslicher Behälter im Rahmen der vorliegenden Erfindung auch Stärke-Derivate geeignet, die durch polymeranaloge Reaktionen aus Stärke erhältlich sind. Solche chemisch modifizierten Stärken umfassen dabei beispielsweise Produkte aus Veresterungen beziehungsweise Veretherungen, in denen Hydroxy-Wasserstoffatome substituiert wurden. Aber auch Stärken, in denen die Hydroxy-Gruppen gegen funktionelle Gruppen, die nicht über ein Sauerstoffatom gebunden sind, ersetzt wurden, lassen sich als Stärke-Derivate einsetzen. In die Gruppe der Stärke-Derivate fallen beispielsweise Alkalistärken, Carboxymethylstärke (CMS), Stärkeester und -ether sowie Aminostärken.

Reine Cellulose weist die formale Bruttozusammensetzung (C₆H₁₀0₅), auf und stellt formal betrachtet ein ß-1,4-Polyacetal von Cellobiose dar, die ihrerseits aus zwei Molekülen Glucose aufgebaut ist. Geeignete Cellulosen bestehen dabei aus ca. 500 bis 5.000 Glucose-Einheiten und haben demzufolge durchschnittliche Molmassen von 50.000 bis 500.000 g/mol. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen beziehungsweise Veretherungen, in denen Hydroxy-Wasserstoffatome substituiert wurden. Aber auch Cellulosen, in denen die Hydroxy-Gruppen gegen funktionelle Gruppen, die nicht über ein Sauerstoffatom gebunden sind, ersetzt wurden, lassen sich als Cellulose-Derivate einsetzen. In die Gruppe der Cellulose-Derivate fallen beispielsweise Alkalicellulosen, Carboxymethylcellulose (CMC), Celluloseester und -ether sowie Aminocellulosen.

Das wasserlösliche Material kann weitere Additive aufweisen. Hierbei handelt es sich beispielsweise um Weichmacher, wie beispielsweise Dipropylenglycol, Ethylenglycol oder Diethylenglycol, Wasser oder Aufschlussmittel.

Besonders bevorzugt wird Polyvinylalkohol als wasserlösliches Material eingesetzt. Dieses ist einerseits leicht zu verarbeiten und kostengünstig zu erhalten. Zudem ist es besonders gut in Wasser löslich und ermöglicht so vielfältige Einsatzmöglichkeiten des hergestellten Behälters.

Es ist erfindungsgemäß bevorzugt, wenn in dem wasserlöslichen Material zur Erhöhung der Produktsicherheit mindestens ein Bittermittel enthalten ist.

Bevorzugte Bittermittel weisen einen Bitterwert von mindestens 1.000, bevorzugt mindestens 10.000, besonders bevorzugt mindestens 200.000 auf. Zur Bestimmung des Bitterwertes wird das im Europäischen Arzneibuch (5. Ausgabe Grundwerk, Stuttgart 2005, Band 1 Allgemeiner Teil Monografiegruppen, 2.8.15 Bitterwert S. 278) beschriebene standardisierte Verfahren verwendet. Als Vergleich dient eine wässrige Lösung von Chininhydrochlorid, dessen Bitterwert mit 200.000 festgelegt ist. Dies bedeutet, dass 1 Gramm Chininhydrochlorid 200 Liter Wasser bitter macht. Die interindividuellen Geschmacksunterschiede bei der organoleptischen Prüfung der Bitterkeit werden bei diesem Verfahren durch einen Korrekturfaktor ausgeglichen.

Ganz besonders bevorzugte Bittermittel werden ausgewählt aus Denatoniumbenzoat, Glycosiden, Isoprenoiden, Alkaloiden, Aminosäuren und Mischungen daraus, besonders bevorzugt Denatoniumbenzoat.

Glycoside sind organische Verbindungen der allgemeinen Struktur R-O-Z, bei denen ein Alkohol (R-OH) über eine glycosidische Bindung mit einem Zuckerteil (Z) verbunden ist.

Geeignete Glycoside sind beispielsweise Flavonoide wie Quercetin oder Naringin oder Iridoid, wie Aucubin und insbesondere Secoiridoid, wie Amarogentin, Dihydrofoliamentin, Gentiopikrosid, Gentiopikrin, Swertiamarin, Swerosid, Gentioflavosid, Centaurosid, Methiafolin, Harpagosid und Centapikrin, Sailicin oder Kondurangin.

Isoprenoide sind Verbindungen, die sich formal von Isopren ableiten. Beispiele sind insbesondere Terpene und Terpenoide.

Geeignete Isoprenoide umfassen beispielsweise Sequiterpenlactone wie Absinthin, Artabsin, Cnicin, Lactucin, Lactucopikrin oder Salonitenolid, Monoterpen-Ketone (Thujone) wie beispielsweise α-Thujon oder β-Thujon, Tetranortriterpene (Limonoide) wie Desoxylimonen, Desoxylimonensäure, Limonin, Ichangin, Iso-Obacunonsäure, Obacunon, Obacunonsäure, Nomilin oder Nomilinsäure, Terpene wie Marrubin, Prämarrubin, Carnosol, Carnosolsäure oder Quassin.

Alkaloide bezeichnen natürlich vorkommende, chemisch heterogene, meist alkalische, stickstoffhaltige organische Verbindungen des Sekundärstoffwechsels, die auf den tierischen oder menschlichen Organismus wirken.

Geeignete Alkaloide sind beispielsweise Chininhydrochlorid, Chininhydrogensulfat, Chinindihydrochlorid, Chininsulfat, Columbin und Coffein.

Geeignete Aminosäuren umfassen beispielsweise Threonin, Methionin, Phenylalanin, Tryptophan, Arginin, Histidin, Valin und Asparaginsäure.

Besonders bevorzugte Bitterstoffe sind Chininsulfat (Bitterwert = 10.000), Naringin (Bitterwert = 10.000), Saccharoseoctaacetat (Bitterwert = 100.000), Chininhydrochlorid, Denatoniumbenzoat (Bitterwert > 100.000.000) und Mischungen daraus, ganz besonders bevorzugt Denatoniumbenzoat (z.B. erhältlich als Bitrex^{®}).

Das wasserlösliche Material enthält bezogen auf dessen Gesamtgewicht bevorzugt Bittermittel (besonders bevorzugt Denatonium Benzoat) in einer Gesamtmenge von höchstens 1 Gewichtsteil Bitterstoff zu 250 Gewichtsteilen viskoelastischer, festförmiger Zusammensetzung (1 : 250), besonders bevorzugt von höchstens 1 : 500, ganz besonders bevorzugt von höchstens 1 : 1000.

Dabei ist es bevorzugt, die Zusammensetzung zum Waschen von Textilien, insbesondere zur Entfernung von Anschmutzungen, die auf Bestandteilen und Rückständen von Deodorantien, Rost, Beeren, Tee und Rotwein basieren, zu verwenden.

Es kann, wie bereits erwähnt, die Anschmutzung mit der erfindungsgemäßen Zusammensetzung dem eigentlichen Waschverfahren vorbehandelt werden und/oder zunächst eine tensidhaltige Flotte als Waschlösung bereitgestellt wird, die das erfindungsgemäße Mehrkomponenten-Waschmittel enthält und die anschließend mit dem zu reinigendem Textil in Kontakt gebracht wird. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung der Zusammensetzung behandelt wird. In den beschriebenen Waschverfahren werden in verschiedenen Ausführungsformen der Erfindung Temperaturen von 60°C oder weniger, beispielsweise 40°C oder weniger, eingesetzt. Diese Temperaturangaben beziehen sich auf die in den Waschschritten eingesetzten Temperaturen.

Es ist ferner bevorzugt, das erfindungsgemäße Verfahren in einer Waschmaschine durchzuführen. Dabei hat es sich als besonders wirksam und daher bevorzugt herausgestellt, wenn die besagte Zusammensetzung in die Trommel einer Waschmaschine dosiert wird.

Alle hierin im Zusammenhang mit den Waschmitteln der Erfindung beschriebenen Ausführungsformen, insbesondere im Hinblick auf die Spezifikation der Inhaltsstoffe, sind gleichermaßen auf die beschriebenen Verfahren und Verwendungen anwendbar und umgekehrt

### Beispiele

### 1.0 Bereitstellung der Testzusammensetzungen

Es wurden folgende flüssige Waschmittel hergestellt:

| Inhaltsstoff | V1 (Vergleich) Gew.-% (Aktivsubstanz) | V2 (Vergleich) Gew.-% (Aktivsubstanz) | E1 (Erfindung) Gew.-% (Aktivsubstanz) |
|---|---|---|---|
| Propylenglykol | 8,2 | 8,2 | 8,2 |
| Glycerin | 10,5 | 10,5 | 10,5 |
| Optischer Aufheller | 0,6 | 0,6 | 0,6 |
| Lineares Alkylbenzolsulfonat | 22,0 | 22,0 | 22,0 |
| C₁₃₋₁₅ Oxoalkohol mit 8 EO Einheiten | 24,0 | 24,0 | 24,0 |
| 2-Aminoethanol | 6,0 | 6,0 | 6,0 |
| C₁₂₋₁₈ Seife- Natriumsalz | 7,5 | 7,5 | 7,5 |
| Ethoxyliertes Polyethylenimin Polymer (PEI 600 mit 20 EO) | 6,0 | 6,0 | 6,0 |
| Diethylenetriaminepenta(methylenphosphonsäure)-Heptanatrium-salz (DTPMPA-7 Na-Salz) | 0,7 | 0,7 | 0,7 |
| Ethanol | 3,0 | 3,0 | 3,0 |
| N,N'-Dipropyl-2,3-dihydroxyterephthaldiamid ¹ | 0,5 | 0,5 | 0,5 |
| Soil Release Polymer (Tephthalsäure-Polyester) | 1,4 | 1,4 | 1,4 |
| Parfüm | 1,7 | 1,7 | 1,7 |
| Catechol-Derivat ¹ | 1,5 | - | 1,5 |
| Mannanase gemäß SEQ ID No.1 ² | - | 0,04 | 0,04 |
| Wasser | auf 100 | auf 100 | auf 100 |

| | | | |
|---|---|---|---|
| ¹ erfindungsgemäße Catecholverbindung der Formel (I) mit R¹ und R² gleich n-Propyl, hergestellt unter Einsatz von n-Propylamin analog der Vorschrift des Beispiels 2 der WO 2016/074936 A1. ² Sequenzidentität zu dem Polypeptid der Positionen 31 bis 490 gemäß SEQ ID No.1 | | | |

### 2.0 Waschtest

Es wurden Waschversuche bei 40°C mit den in obiger Tabelle angegebenen Rezepturen V1, V2 und E1 (Dosierung 25g auf 17 I Wasser von 16 °dH) mit standardisierter "Mousse au chocolat"-Anschmutzung auf Baumwolle durchgeführt.

Vor dem Waschen und nach Trocknung der gewaschenen Baumwoll-Lappen wurde deren Helligkeit mit Hilfe der Farbabstandsmessung gemäß der L*a*b*-Werte bestimmt und die Y-Werte als Maß für die Helligkeit bestimmt. Je größer der Y-Wert umso näher liegt der Wert des Waschguts nach der Wäsche am Ausgangswert ohne Schmutz.

Nachfolgende Tabelle zeigt die ermittelten Y-Werte.

### Y Werte nach dem Waschversuch:

| Anschmutzung | V1 | V2 | E1 |
|---|---|---|---|
| Mousse au chocolat | 47,9 | 48,8 | 50,2 |

## Patentansprüche

1. Tensidzusammensetzung, insbesondere Textilwaschmittel, welche
- mindestens eine Catecholverbindung der Formel (I) 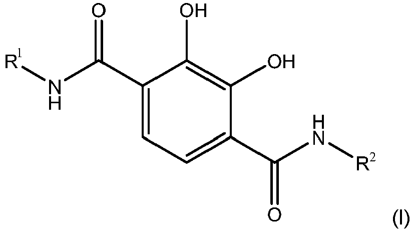 wobei
R¹ und R² unabhängig voneinander für einen Kohlenwasserstoffrest mit 1 bis 20 Kohlenstoffatomen stehen, der gegebenenfalls mit mindestens einem Rest substituiert ist, ausgewählt aus Hydroxy, (C₁-C₄)-Alkoxy, (C₁-C₄)-Alkoxy(CH₂CH₂O)ₙ-, -NR'R" oder -N⁺R'R"R‴ X, wobei n = 1 bis 10, R', R" und R‴ unabhängig voneinander für H oder einen linearen oder verzweigten aliphatischen Kohlenwasserstoffrest mit 1 bis 3, vorzugsweise 1 bis 2 Kohlenstoffatomen und X⁻ für ein Anion stehen;
- mindestens ein Tensid, bevorzugt bezogen auf das Gesamtgewicht der Tensidzusammensetzung in einer Gesamtmenge von 2 bis 70 Gew.-%, insbesondere von 10 bis 65 Gew.-%, besonders bevorzugt von 15 bis 60 Gew.-%;
- mindestens ein Mannanase-Enzym;
- gegebenenfalls weitere Aktivstoffe; und
- Wasser bezogen auf das Gesamtgewicht der Tensidzusammensetzung von 1 bis 50 Gew.%, bevorzugt 2 bis 30 Gew.%, besonders bevorzugt 3 bis 25 Gew.%, ganz besonders bevorzugt 5 bis 25 Gew.%, insbesondere 5 bis 35 Gew.%, bevorzugt 5 bis 30 Gew.%, besonders bevorzugt 5 bis 25 Gew.%, ganz besonders bevorzugt 8 bis 15 Gew.%, umfasst.

2. Tensidzusammensetzung nach Anspruch 1, **dadurch gekennzeichnet, dass** in Formel (I) die Reste R¹ und R² unabhängig voneinander für eine Alkylgruppe, eine Alkoxyalkylgruppe, eine Hydroxyalkylgruppe, eine Hydroxyalkyloxyalkyl-Gruppe, (N-Hydroxyethyl)-aminoethyl, (N-Methoxyethyl)-aminoethyl oder (N-Ethoxyethyl)-aminoethyl, oder eine aromatische Gruppe stehen.

3. Tensidzusammensetzung nach einem der Ansprüche 1 oder 2, **dadurch gekennzeichnet, dass** in Formel (I) die Reste R¹ und R² gleich sind.

4. Tensidzusammensetzung nach einem der Ansprüche 1 bis 3, **dadurch gekennzeichnet, dass** bezogen auf deren Gesamtgewicht die mindestens eine Catecholverbindung der Formel (I) in einer Gesamtmenge von 0,2 bis 90 Gew.%, bevorzugt 1 bis 85 Gew.%, insbesondere 2,5 bis 75 Gew.-%, enthalten ist.

5. Tensidzusammensetzung nach einem der Ansprüche 1 bis 4, **dadurch gekennzeichnet, dass** mindestens ein anionisches Tensid und/oder mindestens ein nichtionisches Tensid enthalten ist.

6. Tensidzusammensetzung nach einem der Ansprüche 1 bis 5, **dadurch gekennzeichnet, dass** bezogen auf deren Gesamtgewicht Mannanase-Enzym in einer Gesamtmenge von 0,01 bis 2,5 Gew.-%, insbesondere von 0,02 bis 1,0 Gew.-%, enthalten ist.

7. Tensidzusammensetzung nach einem der Ansprüche 1 bis 6, **dadurch gekennzeichnet, dass** das Mannanase-Enzym mindestens ein Mannanase-Polypeptid aus gram-positiven alkalophilen Stämmen von *Bacillus* enthält, insbesondere ausgewählt aus mindestens einem Vertreter der Gruppe aus *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis, and Bacillus sp., besonders bevorzugt ausgewählt aus mindestens einem Vertreter der Gruppe aus Bacillus sp. 1633, Bacillus sp. AA112, Bacillus clausii, Bacillus agaradhaerens and Bacillus licheniformis.*

8. Tensidzusammensetzung nach einem der Ansprüche 1 bis 7, **dadurch gekennzeichnet, dass** das Mannanase-Enzym ausgewählt wird aus mindestens einem Vertreter aus der Gruppe, die gebildet wird aus Polypeptiden, die eine Aminosäuresequenz umfassen, die mindestens 90%, zunehmend bevorzugt mindestens 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% oder 99,8%, Sequenzidentität zu dem Polypeptid der Positionen 31 bis 490 gemäß SEQ ID No.1 aufweisen.

9. Tensidzusammensetzung nach einem der Ansprüche 1 bis 8, **dadurch gekennzeichnet, dass** sie enthält
• mindestens eine Mannanase aus *Paenibacillus sp.* oder
• eine der Varianten davon, umfassend eine Aminosäuresequenz, die mindestens zwei Veränderungen aufweist, die ausgewählt sind aus
(i) mindestens einer Substitution an mindestens einer der Positionen, die aus 1, 2, 3, 4, 6, 10, 19, 28, 30, 38, 59, 60, 61, 62, 63, 66, 67, 68, 70, 71, 74, 75, 78, 80, 82, 93, 97, 103, 111, 124, 129, 131, 135, 136, 139, 143, 150, 167, 168, 184, 213, 214, 217, 225, 228, 235, 242, 244, 258, 259, 261, 283 und 284 ausgewählt sind, und
(ii) einer Insertion an Position 298, wobei die genannte Aminosäurepositionen den Positionen gemäß SEQ ID No. 2 dieser Anmeldung. SEQ ID No. 2 entsprechen.

10. Verfahren zum Waschen von Textilien, umfassend die Schritte,
Zugeben einer Tensidzusammensetzung nach einem der Ansprüche 1 bis 9 und Durchführen einer Waschprozedur, bevorzugt in einer Waschmaschine.

## Claims

1. Surfactant composition, in particular textile detergent, comprising
- at least one catechol compound of formula (I) 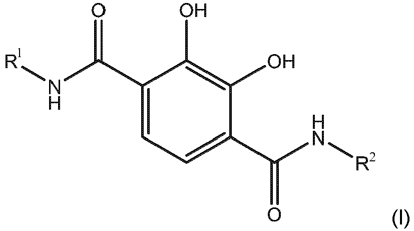 wherein
R¹ and R² independently of one another represent a hydrocarbon radical having 1 to 20 carbon atoms which is optionally substituted by at least one radical selected from hydroxyl, (C₁-C₄)-alkoxy, (C₁-C₄)-alkoxy(CH₂CH₂O)ₙ -, -NR'R" or -N⁺R'R"R‴ X⁻, where n = 1 to 10, R', R" and R‴ independently of one another represent H or a linear or branched aliphatic hydrocarbon radical having 1 to 3, preferably 1 to 2, carbon atoms and X⁻ represents an anion;
- at least one surfactant, preferably in a total amount of from 2 to 70% by weight, in particular from 10 to 65% by weight, particularly preferably from 15 to 60% by weight, based on the total weight of the surfactant composition;
- at least one mannanase enzyme;
- optionally, further active substances; and
- water based on the total weight of the surfactant composition of 1 to 50 wt.%, preferably 2 to 30 wt.%, more preferably 3 to 25 wt.%, most preferably 5 to 25 wt.%, in particular 5 to 35 wt.%, preferably 5 to 30 wt.%, more preferably 5 to 25 wt.%, most preferably 8 to 15 wt.%.

2. Surfactant composition according to claim 1, **characterized in that** in formula (I), R¹ and R² are independently an alkyl group, an alkoxyalkyl group, a hydroxyalkyl group, a hydroxyalkyloxyalkyl group, (N-hydroxyethyl)aminoethyl, (N-methoxyethyl)aminoethyl or (N-ethoxyethyl)aminoethyl, or an aromatic group.

3. Surfactant composition according to one of claims 1 or 2, **characterized in that** in formula (I) the radicals R¹ and R² are identical.

4. A surfactant composition according to any one of claims 1 to 3, **characterized in that**, based on its total weight, the at least one catechol compound of formula (I) is present in a total amount of 0.2 to 90% by weight, preferably 1 to 85% by weight, in particular 2.5 to 75% by weight.

5. Surfactant composition according to any one of claims 1 to 4, **characterized in that** at least one anionic surfactant and/or at least one nonionic surfactant is present.

6. Surfactant composition according to any one of claims 1 to 5, **characterized in that**, based on its total weight, mannanase enzyme is present in a total amount of from 0.01 to 2.5% by weight, in particular from 0.02 to 1.0% by weight.

7. Surfactant composition according to any one of claims 1 to 6, **characterized in that** the mannanase enzyme comprises at least one mannanase polypeptide from gram-positive alkalophilic strains of *Bacillus,* in particular selected from at least one member of the group consisting of *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis,* and *Bacillus sp.,* particularly preferably selected from at least one member of the group consisting of *Bacillus sp. 1633, Bacillus sp. AAI12, Bacillus clausii, Bacillus agaradhaerens* and *Bacillus licheniformis.*

8. A surfactant composition according to any one of claims 1 to 7, **characterized in that** the mannanase enzyme is selected from at least one member of the group consisting of polypeptides comprising an amino acid sequence having at least 90%, increasingly preferably at least 90.5%, 91%, 91.5%, 92%, 92.5%, 93%, 93.5%, 94%, 94.5%, 95%, 95.5%, 96%, 96.5%, 97%, 97.5%, 98%, 98.5%, 99.0%, 99.1%, 99.2%, 99.3%, 99.4%, 99.5%, 99.6%, 99.7% or 99.8%, sequence identity to the polypeptide of positions 31 to 490 according to SEQ ID No.1.

9. Surfactant composition according to any one of claims 1 to 8, **characterized in that** it contains
• at least one mannanase from *Paenibacillus sp.* or
• one of the variants thereof comprising an amino acid sequence having at least two modifications selected from
(i) at least one substitution at at least one of the positions selected from 1, 2, 3, 4, 6, 10, 19, 28, 30, 38, 59, 60, 61, 62, 63, 66, 67, 68, 70, 71, 74, 75, 78, 80, 82, 93, 97, 103, 111, 124, 129, 131, 135, 136, 139, 143, 150, 167, 168, 184, 213, 214, 217, 225, 228, 235, 242, 244, 258, 259, 261, 283 and 284, and
(ii) an insertion at position 298, said amino acid positions corresponding to the positions according to SEQ ID No. 2 of this application.

10. Method of washing textiles comprising the steps of,
Adding a surfactant composition according to any one of claims 1 to 9 and
Performing a washing procedure, preferably in a washing machine.

## Revendications

1. Composition tensioactive, en particulier détergent pour textiles, qui comprend
- au moins un composé catéchol de formule (I) 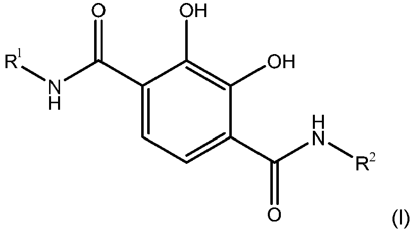 où
R¹ et R² représentent, indépendamment l'un de l'autre, un radical hydrocarboné ayant de 1 à 20 atomes de carbone, qui est éventuellement substitué par au moins un radical choisi parmi les radicaux hydroxy, (C₁-C₄)-alcoxy, (C₁-C₄)-alcoxy(CH₂CH₂O)ₙ-, -NR'R" ou -N⁺R'R"R‴ X⁻ , où n = 1 à 10, R' , R" et R‴ représentent indépendamment les uns des autres H ou un radical hydrocarboné aliphatique linéaire ou ramifié ayant de 1 à 3, de préférence de 1 à 2 atomes de carbone, et X⁻ représente un anion;
- au moins un tensioactif, de préférence par rapport au poids total de la composition de tensioactifs, en une quantité totale de 2 à 70 % en poids, en particulier de 10 à 65 % en poids, de manière particulièrement préférée de 15 à 60 % en poids;
- au moins une enzyme mannanase;
- d'autres substances actives, le cas échéant; et
- de l'eau par rapport au poids total de la composition d'agents tensioactifs de 1 à 50 % en poids, de préférence de 2 à 30 % en poids, de manière particulièrement préférée de 3 à 25 % en poids, de manière tout à fait préférée de 5 à 25 % en poids, en particulier de 5 à 35 % en poids, de préférence de 5 à 30 % en poids, de manière particulièrement préférée de 5 à 25 % en poids, de manière tout à fait préférée de 8 à 15 % en poids.

2. Composition tensioactive selon la revendication 1, **caractérisée en ce que** dans la formule (I), les radicaux R¹ et R² représentent indépendamment un groupe alkyle, un groupe alcoxyalkyle, un groupe hydroxyalkyle, un groupe hydroxyalkyloxyalkyle, un groupe (N-hydroxyéthyl)-aminoéthyle, un groupe (N-méthoxyéthyl)-aminoéthyle ou un groupe (N-éthoxyéthyl)-aminoéthyle, ou un groupe aromatique.

3. Composition tensioactive selon l'une quelconque des revendications 1 ou 2, **caractérisée en ce que**, dans la formule (I), les radicaux R¹ et R² sont identiques.

4. Composition tensioactive selon l'une quelconque des revendications 1 à 3, **caractérisée en ce que**, par rapport à son poids total, ledit au moins un composé catéchol de formule (I) est contenu en une quantité totale de 0,2 à 90 % en poids, de préférence de 1 à 85 % en poids, en particulier de 2,5 à 75 % en poids.

5. Composition tensioactive selon l'une quelconque des revendications 1 à 4, **caractérisée en ce qu'**elle comprend au moins un tensioactif anionique et/ou au moins un tensioactif non ionique.

6. Composition tensioactive selon l'une quelconque des revendications 1 à 5, **caractérisée en ce que**, par rapport à son poids total, l'enzyme mannanase est contenue en une quantité totale de 0,01 à 2,5 % en poids, en particulier de 0,02 à 1,0 % en poids.

7. Composition tensioactive selon l'une quelconque des revendications 1 à 6, **caractérisée en ce que** l'enzyme mannanase comprend au moins un polypeptide mannanase issu de souches alcalophiles gram-positives de *Bacillus,* notamment choisi parmi au moins un représentant du groupe constitué par *Bacillus subtilis, Bacillus lentus, Bacillus clausii, Bacillus agaradhaerens, Bacillus brevis, Bacillus stearothermophilus, Bacillus alkalophilus, Bacillus amyloliquefaciens, Bacillus coagulans, Bacillus circulans, Bacillus lautus, Bacillus thuringiensis, Bacillus cheniformis,* et *Bacillus sp.,* de préférence au moins un membre du groupe constitué de *Bacillus sp. I633, Bacillus sp. AAI12, Bacillus clausii, Bacillus agaradhaerens* et *Bacillus licheniformis.*

8. Composition tensioactive selon l'une quelconque des revendications 1 à 7, **caractérisée en ce que** l'enzyme mannanase est choisie parmi au moins un membre du groupe formé par les polypeptides comprenant une séquence d'acides aminés représentant au moins 90%, de plus en plus de préférence au moins 90,5%, 91%, 91,5%, 92%, 92,5%, 93%, 93,5%, 94%, 94,5%, 95%, 95,5%, 96%, 96,5%, 97%, 97,5%, 98%, 98,5%, 99,0%, 99,1%, 99,2%, 99,3%, 99,4%, 99,5%, 99,6%, 99,7% ou 99,8%, d'identité de séquence avec le polypeptide des positions 31 à 490 selon SEQ ID NO.1.

9. Composition tensioactive selon l'une quelconque des revendications 1 à 8, **caractérisée en ce qu'**elle contient
• au moins une mannanase de *Paenibacillus sp.* ou
• l'une de ses variantes comprenant une séquence d'acides aminés ayant au moins deux changements choisis parmi
(i) au moins une substitution en au moins une des positions choisies parmi 1, 2, 3, 4, 6, 10, 19, 28, 30, 38, 59, 60, 61, 62, 63, 66, 67, 68, 70, 71, 74, 75, 78, 80, 82, 93, 97, 103, 111, 124, 129, 131, 135, 136, 139, 143, 150, 167, 168, 184, 213, 214, 217, 225, 228, 235, 242, 244, 258, 259, 261, 283 et 284, et
(ii) une insertion en position 298, lesdites positions d'acides aminés correspondant aux positions selon SEQ ID No. 2 de la présente demande.

10. Procédé de lavage de textiles, comprenant les étapes consistant à
ajouter une composition de tensioactifs selon l'une quelconque des revendications 1 à 9, et
exécution d'une procédure de lavage, de préférence dans une machine à laver.
